(19) Europäisches Patentamt
European Patent Office
Office européen des brevets

(11) EP 2 791 154 B1

(12) # EUROPÄISCHE PATENTSCHRIFT

(45) Veröffentlichungstag und Bekanntmachung des Hinweises auf die Patenterteilung:
**31.08.2016 Patentblatt 2016/35**

(21) Anmeldenummer: **12808314.4**

(22) Anmeldetag: **12.12.2012**

(51) Int Cl.:
***C07F 11/00*** *(2006.01)* ***C08G 18/48*** *(2006.01)*
***C08G 18/76*** *(2006.01)* ***C08G 18/22*** *(2006.01)*
***C09J 175/08*** *(2006.01)* ***C09D 175/08*** *(2006.01)*

(86) Internationale Anmeldenummer:
**PCT/EP2012/075202**

(87) Internationale Veröffentlichungsnummer:
**WO 2013/087679 (20.06.2013 Gazette 2013/25)**

(54) **DIOXOMOLYBDÄN(VI)-KOMPLEXVERBINDUNGEN ALS KATALYSATOREN FÜR POLYURETHAN-ZUSAMMENSETZUNGEN**

DIOXOMOLYBDENUM(VI) COMPLEXES AS CATALYSTS FOR POLYURETHANE COMPOSITIONS

COMPOSÉS À BASE DE COMPLEXES DE DIOXOMOLYBDÈNE (VI) COMME CATALYSEURS DE COMPOSITIONS DE POLYURÉTHANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.12.2011 EP 11193060**

(43) Veröffentlichungstag der Anmeldung:
**22.10.2014 Patentblatt 2014/43**

(73) Patentinhaber: **Sika Technology AG**
**6340 Baar (CH)**

(72) Erfinder:
- **BURCKHARDT, Urs**
**CH-8049 Zürich (CH)**
- **CANNAS, Rita**
**CH-8600 Dübendorf (CH)**

(74) Vertreter: **Sika Patent Attorneys**
**c/o Sika Technology AG**
**Corp. IP Dept.**
**Tüffenwies 16**
**Postfach**
**8048 Zürich (CH)**

(56) Entgegenhaltungen:
**WO-A1-2007/003966 DE-A1- 10 131 463**

- **CHRISTENSEN KENNER A ET AL: "Molybdenum-95 NMR spectra of dioxomolybdenum(VI) complexes", INORGANICA CHIMICA ACTA, ELSEVIER BV, NL, Bd. 56, Nr. 2, 1. Januar 1981 (1981-01-01) , Seiten L27-L28, XP009159254, ISSN: 0020-1693, DOI: 10.1016/S0020-1693(00)88521-7 [gefunden am 2001-04-17]**
- **ABRAMENKO V L ET AL: "Synthesis and IR spectral study of MoO2Cl2 molecular complexes with acetoacetanilides. Crystal structure of MoO2Cl2 complex with acetoacet-2-toluidine", RUSSIAN JOURNAL OF COORDINATION CHEMISTRY, CONSULTANTS BUREAU, NEW YORK, NY, US, Bd. 26, Nr. 12, 1. Dezember 2000 (2000-12-01), Seiten 866-871, XP009159253, ISSN: 1070-3284, DOI: 10.1023/A:1026679009037 [gefunden am 2004-11-04]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 791 154 B1

## Beschreibung

### Technisches Gebiet

Die vorliegende Erfindung betrifft das Gebiet der Polyurethan-Zusammensetzungen sowie Katalysatoren für Polyurethan-Zusammensetzungen.

### Stand der Technik

Polyurethan-Zusammensetzungen sind seit langem bekannt und werden in vielen Bereichen eingesetzt. Klassisch wird in der Fachwelt zwischen einkomponentigen und zweikomponentigen Polyurethan-Zusammensetzungen unterschieden. Einkomponentige Polyurethan-Zusammensetzungen härten unter dem Einfluss von Luftfeuchtigkeit aus. Zweikomponentige Polyurethan-Zusammensetzungen enthalten als zweite Komponente eine Härterkomponente, welche im Wesentlichen Polyamine und/oder Polyole enthält. In beiden Fällen werden Isocyanatgruppen-haltige Verbindungen oder Prepolymere eingesetzt.

Um die Aushärtung zu beschleunigen, werden Katalysatoren beigefügt. Zwar ist eine Vielzahl von Polyurethan-Katalysatoren bekannt, allerdings ist eine Großzahl hinsichtlich der Urethanisierungsreaktion, d.h. der Reaktion von alkoholischen OH-Gruppen mit Isocyanatgruppen, nicht sonderlich selektiv, sondern katalysiert mehr oder weniger auch andere Reaktionen der Isocyanatgruppe, wie Allophanat- und Biuret-Bildung oder Cyclotrimerisierung. Insbesondere steht die Urethanisierungsreaktion meist in Konkurrenz zur Reaktion der Isocyanatgruppen mit Wasser, welche unter Freisetzung von gasförmigem Kohlendioxid zu Harnstoffgruppen führt. Diese Nebenreaktion ist bei vielen Polyurethan-Zusammensetzungen, insbesondere bei deren Anwendung als Kleb- und Dichtstoff, als Beschichtung oder Gießharz, störend, da sie bei der Aushärtung zu Blasenbildung und damit schlechter Formstabilität, geringerer Haftung, tieferer mechanischer Festigkeit, unbefriedigender Ästhetik sowie zu wenig reproduzierbaren Ergebnissen führt. Das für die Blasenbildung verantwortliche Wasser stammt entweder aus dem Restwasser-Gehalt der Bestandteile der Zusammensetzung, insbesondere der Polyole und der Füllstoffe, welche auch nach Trocknungsprozessen mehr oder weniger feucht sind und einen typischen Restwasser-Gehalt von 0,01 bis 0,5 Gew.-% aufweisen, oder aus der Umgebungsfeuchtigkeit, welche durch Diffusion aus der Luft oder aus den Substraten in die Zusammensetzung eindringt, was besonders bei hoher Luftfeuchtigkeit, porösen Substraten und/oder hydrophilen Polyolen, wie den in der Praxis oft eingesetzten Polyetherpolyolen, auftritt. Gerade die in der Praxis vielfach verwendeten Aminkatalysatoren, beispielsweise tertiäre Amine, und Zinnkatalysatoren, beispielsweise Dialkylzinncarboxylate, führen oft zu ausgeprägter Blasenbildung. Der Restwasser-Gehalt in der Polyurethan-Zusammensetzung bewirkt außerdem, dass hydrolyseempfindliche Katalysatoren, wie beispielsweise Bismutcarboxylate, bei längerem Aufbewahren der Zusammensetzung vor dem Gebrauch (Lagerung) deaktiviert werden, was sich negativ auf die Aushärtungsgeschwindigkeit und die mechanischen Eigenschaften auswirkt. Bei einigen bekannten Katalysatoren, beispielsweise den Dialkylzinncarboxylaten, ist darüber hinaus die Beständigkeit der ausgehärteten Zusammensetzung unter thermischer Belastung ungenügend, wobei der Katalysator einen Molekulargewichts-Abbau, d.h. eine Depolymerisation, unter Verlust an mechanischer Festigkeit verursacht. Weiterhin sind viele der bekannten Katalysatoren bei Raumtemperatur fest und in den Polyurethan-Ausgangsmaterialien oder den Weichmachern wenig löslich, so dass für ihren Einsatz in Zusammensetzungen, die bei Raumtemperatur härten, organische Lösungsmittel eingesetzt werden müssen. Schließlich sind manche der bekannten Katalysatoren, insbesondere solche auf Basis von Schwermetallverbindungen, toxikologisch bedenklich.

Die Verwendung von Dioxomolybdän(VI)-Komplexen als Katalysatoren für härtbare Massen, beispielsweise Polyurethan-Zusammensetzungen, ist bekannt. So beschreibt die DE 101 31 463 A1 hitzehärtbare Zweikomponenten-Beschichtungssysteme, die unter anderem Dioxomolybdän(VI)- oder Dioxowolfram(VI)-Komplexe von 1,3-Diketon- oder 1,3-Ketoester-Anionen als Liganden enthalten. Ein bevorzugter Ligand ist Acetylacetonat. Die DE 103 08 104 offenbart Einkomponenten-Polyurethan-Beschichtungssysteme auf Basis blockierter Isocyanate, welche beispielsweise Dioxomolybdän(VI)-Komplexe von Acetylacetonat oder Tetramethylheptan-3,5-dionat enthalten. Die WO 2009/106722 A1 offenbart bei Raumtemperatur härtende Organopolysiloxane, die unter anderem Dioxomolybdän(VI)-Komplexe von 1,3-Diketonen enthalten. Gegenteilig hierzu wird gemäß der DE 10 2005 041 246 auf Molybdän- bzw. Wolfram-Verbindungen verzichtet, da diese in den Beschichtungen zu störenden Verfärbungen führen. Gemäß dieser Offenbarung werden vielmehr Cäsiumverbindungen eingesetzt. Die WO2007/003966 offenbart Zirkonium Komplexe mit einem Liganden vom Ketoamid Typ. Die Verbindungen dienen ebenfalls als Katalysatoren zur Aushärtung härtbarer Massen.

Die bekannten Dioxomolybdän(VI)-Komplexe zeigen jedoch eine vergleichsweise schlechte thermische und hydrolytische Stabilität und hydrolysieren in einem restwasserhaltigen Polyol allmählich, wodurch die katalytische Aktivität nicht bewahrt werden kann. Ferner ist ihre Löslichkeit in Weichmachern oder Polyolen begrenzt, und sie können in bei Raumtemperatur härtenden Systemen nicht ohne Verwendung von flüchtigen organischen Lösungsmitteln (VOC) eingesetzt werden.

**Darstellung der Erfindung**

Aufgabe der vorliegenden Erfindung ist es, die vorstehend beschriebenen Nachteile des Standes der Technik zu beseitigen. Insbesondere besteht die Aufgabe der vorliegenden Erfindung darin, einen Katalysator bereitzustellen, der zu einer Verbesserung nachfolgender Eigenschaften bzw. zu einem ausgewogenen Verhältnis dieser führt.

Der Katalysator soll sich durch eine hohe katalytische Aktivität und Selektivität hinsichtlich der Urethanisierungsreaktion, d.h. der Reaktion von alkoholischen OH-Gruppen mit Isocyanatgruppen, auszeichnen und so einen raschen und durch Feuchtigkeit möglichst wenig gestörten Aufbau eines mechanisch hochwertigen Polyurethan-Polymers aus polyfunktionellen Alkoholen (Polyolen) und Polyisocyanaten ermöglichen. Zum anderen soll der Katalysator eine ausreichende Hydrolyseresistenz besitzen, um unter üblichen Lagerbedingungen, d.h. bei Raumtemperatur oder bei leicht erhöhten Temperaturen, über mehrere Monate in einer restwasserhaltigen Polyol-Zusammensetzung ohne starken Aktivitätsverlust erhalten zu bleiben. Weiterhin soll der Katalysator die thermische Beständigkeit des ausgehärteten Polyurethan-Polymers möglichst wenig herabsetzen. Überdies soll der Katalysator bei Raumtemperatur oder bei leicht erhöhten Temperaturen flüssig sein bzw. in den Polyurethan-Ausgangsmaterialien oder in Weichmachern gut löslich sein, damit er in bei Raumtemperatur härtenden, lösungsmittelfreien Systemen auf einfache Weise eingesetzt werden kann. Letztendlich soll der Katalysator eine möglichst geringe Giftigkeit aufweisen.

Insbesondere soll der Katalysator über gute thermische und hydrolytische Stabilität verfügen und damit in einem restwasserhaltigen Polyol nicht schnell hydrolysieren und so die katalytische Aktivität bewahren, als auch bei Raumtemperatur flüssig sein und/oder eine gute Löslichkeit in Weichmachern oder Polyolen aufweisen, um damit in bei Raumtemperatur härtenden Systemen auf einfache Weise und ohne die Verwendung von flüchtigen organischen Lösungsmitteln (VOC) eingesetzt werden zu können.

Überraschenderweise wurden nun eine Dioxomolybdän(VI)-Komplexverbindung gemäß Anspruch 1 mit den gewünschten Eigenschaften gefunden. Die Dioxomolybdän(VI)-Komplexverbindung besitzt die Formel $MoO_2(L)_x(Y)_{2-x}$, wobei x für 1 oder 2 steht, Y für einen einfach negativ geladenen Liganden steht und L für einen Liganden der Formel (I) steht,

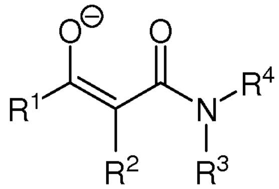

(I)

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und

$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

Der Ligand L der Formel (I) weist formal eine über die 1,3-Ketoamid-Struktur delokalisierte einfach negative Ladung auf. Er kann daher in verschiedenen Grenzstrukturen, beispielsweise in den nachfolgend dargestellten Grenzstrukturen, gezeichnet werden. Alle möglichen Grenzstrukturen des Liganden L der Formel (I) werden im Rahmen der vorliegenden Erfindung als gleichwertig angesehen.

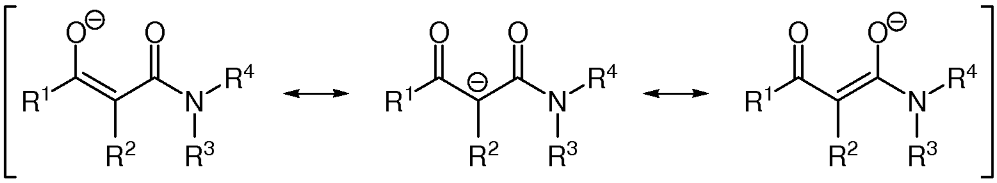

Der Ligand Y stellt einen beliebigen einfach negativ geladenen Liganden dar, insbesondere ein geeignetes organisches Anion, bevorzugt ein Carbonylat, besonders bevorzugt ein 1,3-Dicarbonylat, beispielsweise Acetylacetonat oder 2,2,6,6-Tetramethylheptan-3,5-dionat.

Die erfindungsgemäße Dioxomolybdän(VI)-Komplexverbindung der Formel $MoO_2(L)_x(Y)_{2-x}$ mit Molybdän als Zentralatom und koordinativ gebundenen Liganden L und gegebenenfalls Y ist neutral und enthält einen oder zwei

Liganden L der Formel (I).

Vom Umfang der Erfindung ausgeschlossen ist die Verbindung Dioxomolybdän(VI)-bis(N,N-Diethyl-3-oxobutanamidat), offenbart in Inorganica Chimica Acta, Bd. 56, Nr. 2, Januar 1981, Seiten L27-L28.

Vorzugsweise steht x in der erfindungsgemäßen Dioxomolybdän(VI)-Komplexverbindung der Formel $MoO_2(L)_x(Y)_{2-x}$ für 2, da die Komplexverbindung der Formel $MoO_2(L)_2$ besonders stabil ist. Die beiden Liganden L der Formel (I) können dabei gleich oder verschieden sein. Besonders bevorzugt liegen zwei gleiche Liganden L der Formel (I) vor.

In der Formel (I) stehen $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen.

Bei dem einwertigen gesättigten Kohlenwasserstoff-Rest mit 1 bis 10 Kohlenstoff-Atomen handelt es sich bevorzugt um einen Alkyl-Rest mit 1 bis 4 Kohlenstoffatomen, insbesondere einen Methyl- oder einen Butyl-Rest. Diese haben den Vorteil, dass die Komplexverbindung damit tendenziell flüssig oder gut löslich ist. Bei dem einwertigen ungesättigten Kohlenwasserstoff-Rest handelt es sich bevorzugt auch um einen Arylrest, insbesondere um einen Phenyl-Rest.

Besonders bevorzugt ist $R^2$ ein Wasserstoff-Rest, da die Komplexverbindung damit tendenziell besonders stabil ist.

Unter einem zweiwertigen Alkylen-Rest mit 3 bis 6 Kohlenstoffatomen wird ein Rest der Formel $-(CH_2)_n-$ verstanden, wobei n 3 bis 6 bedeutet.

Bevorzugt bilden $R^1$ und $R^2$ zusammen einen zweiwertigen Alkylen-Rest mit 3 bis 4 Kohlenstoffatomen, insbesondere mit 3 Kohlenstoffatomen.

$R^3$ und $R^4$ stehen unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen.

Bei dem einwertigen gesättigten Kohlenwasserstoff-Rest mit 1 bis 12 Kohlenstoffatomen handelt es sich bevorzugt um einen Alkylrest mit 1 bis 8 Kohlenstoffatomen, besonders bevorzugt um einen Methyl-, einen Ethyl-, einen Propyl-, einen Isopropyl-, einen Butyl-, einen Isobutyl-, einen Hexyl-, einen 2-Methyl-pentyl-, einen Octyl-, oder einen 2-Ethyl-hexyl-Rest. Dies hat den Vorteil, dass die Komplexverbindung damit tendenziell flüssig oder gut löslich ist. Vorzugsweise kann der einwertige gesättigte Kohlenwasserstoff-Rest mit 1 bis 12 Kohlenstoffatomen auch ein Cycloalkyl-Rest mit 5 bis 6 Kohlenstoffatomen, besonders bevorzugt 6 Kohlenstoffatomen, sein. Bei dem einwertigen gesättigten Kohlenwasserstoff-Rest mit Heteroatomen handelt es sich vorzugsweise um einen Hydroxyalkyl-Rest mit 1 bis 4 Kohlenstoffatomen, besonders bevorzugt um einen 2-Hydroxyethyl- oder 2-Hydroxy-propyl-Rest. Dies hat den Vorteil, dass die Komplexverbindung damit tendenziell flüssig oder gut löslich ist und der Ligand bei der Aushärtung in das Polymer kovalent eingebunden werden kann. Bevorzugt ist auch ein Alkylether-Rest mit 1 bis 4 Kohlenstoffatomen, besonders bevorzugt ein 2-Methoxyethyl- oder ein 2-(2-Methoxy)ethoxyethyl-Rest, da die Komplexverbindung damit tendenziell flüssig oder gut löslich ist.

$R^3$ kann zusammen mit $R^4$ vorzugsweise auch einen zweiwertigen Alkylen-Rest der Formel $-(CH_2)_n-X-(CH_2)_n-$ mit X=O, NR, wobei R ein einwertiger Alkyl-Rest mit 1 bis 4 Kohlenstoffatomen ist, oder S und n = 2 bis 4 bilden. Besonders bevorzugt ist n=2 und X=O oder NR.

Die Auswahl der bevorzugten Reste in den Liganden L der Formel (I) beruht vorzugsweise darauf, dass die entsprechenden 1,3-Ketoamide, welche als Ausgangsstoffe zur Herstellung der erfindungsgemäßen Dioxomolybdän(VI)-Komplexverbindung der Formel $MoO_2(L)_x(Y)_{2-x}$ eingesetzt werden, einfach herstellbar und/oder kommerziell erhältlich und damit preisgünstig sind.

Bevorzugt sind Dioxomolybdän(VI)-Komplexverbindungen der Formel $MoO_2(L)_2$ mit zwei gleichen Liganden L der Formel (I), wobei $R^1$ für einen Methyl-Rest, $R^2$ für einen Wasserstoff-Rest und $R^3$ und $R^4$ jeweils für einen Ethyl-Rest stehen.

Besonders bevorzugt sind nachfolgende Dioxomolybdän(VI)-Komplexverbindungen (1) bis (8) der Formel $MoO_2(L)_2$ mit zwei gleichen Liganden L der Formel (I), wobei $R^1$ bis $R^4$ die in der Tabelle genannten Bedeutungen besitzen.

| | $R^1$ | $R^2$ | $R^3$ | $R^4$ |
|---|---|---|---|---|
| (1) | Alkyl-Rest mit 1-4 Kohlenstoffatomen | Wasserstoff-Rest | Alkyl-Rest mit 1-8 Kohlenstoffatomen | Alkyl-Rest mit 1-8-Kohlenstoffatomen |
| (2) | Phenyl-Rest | Wasserstoff-Rest | Alkyl-Rest mit 1-8 Kohlenstoffatomen | Alkyl-Rest mit 1-8-Kohlenstoffatomen |

(fortgesetzt)

| | $R^1$ | $R^2$ | $R^3$ | $R^4$ |
|---|---|---|---|---|
| (3) | Alkyl-Rest mit 1-4 Kohlenstoffatomen | Wasserstoff-Rest | Alkylether-Rest mit 1-4 Kohlenstoffatomen | Alkylether-Rest mit 1-4 Kohlenstoffatomen |
| (4) | Alkylen-Rest mit 3-6 Kohlenstoffatomen | | Alkyl-Rest mit 1-8 Kohlenstoffatomen | |
| (5) | Alkyl-Rest mit 1-4 Kohlenstoffatomen | Wasserstoff-Rest | Alkylen-Rest der Formel $-(CH_2)_n-X-(CH_2)_n-$ mit X=O oder NR und n=2 | |
| (6) | Alkyl-Rest mit 1-4 Kohlenstoffatomen | Wasserstoff-Rest | Cycloalkyl-Rest mit 5-6 Kohlenstoffatomen | Alkyl-Rest mit 1-8 Kohlenstoffatomen |
| (7) | Alkyl-Rest mit 1-4 Kohlenstoffatomen | Wasserstoff-Rest | Alkyl-Rest mit 1-8 Kohlenstoffatomen | Cycloalkyl-Rest mit 5-6 Kohlenstoffatomen |
| (8) | Phenyl-Rest | Wasserstoff-Rest | Alkylen-Rest der Formel $(-CH_2)_n-X-(CH_2)_n-$ mit X=O oder NR und n=2 | |

In einer bevorzugten Ausführungsform handelt es sich bei der erfindungsgemäßen Dioxomolybdän(VI)-Komplexverbindungen der Formel $MoO_2(L)_x(Y)_{2-x}$ nicht um Dioxomolybdän(VI)-bis(N,N-Diethyl-3-oxobutanamidat).

Die Herstellung der erfindungsgemäßen Dioxomolybdän(VI)-Komplexverbindung der Formel $MoO_2(L)_x(Y)_{2-x}$ erfolgt durch Umsetzung eines 1,3-Ketoamids der Formel

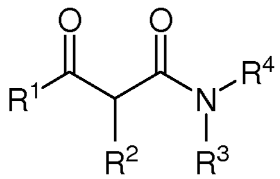

mit $R^1$, $R^2$, $R^3$ und $R^4$, wie vorstehend definiert, mit einem Dioxomolybdän(VI)-Salz oder -Komplex. Bevorzugt ist der Einsatz von Molybdat-Alkalimetallsalzen, wie Natriummolybdat-dihydrat, und der Einsatz von Dioxomolybdän(VI)-bis(acetylacetonat).

Das 1,3-Ketoamid kann dabei stöchiometrisch oder überstöchiometrisch eingesetzt werden. Bei einem überstöchiometrischen Einsatz des 1,3-Ketoamids weist die erfindungsgemäße Dioxomolybdän(VI)-Komplexverbindung tendenziell eine erhöhte Hydrolysestabilität und eine niedrigere Viskosität auf. Bevorzugt liegt das stöchiometrische Verhältnis zwischen dem Dioxomolybdän(VI)-Salz oder -Komplex und dem 1,3-Ketoamid im Bereich von 1:2 bis 1:4.

Das/der vorzugsweise getrocknete Dioxomolybdän(VI)-Salz oder -Komplex wird mit dem 1,3-Ketoamid vermischt und die Mischung vorzugsweise unter Rühren während 1 bis 24 Stunden, bevorzugt etwa 2 Stunden, auf eine Temperatur von 50 bis 130 °C, insbesondere von etwa 80 °C erwärmt. Anschließend wird das Reaktionsgemisch vorzugsweise im Vakuum von flüchtigen Bestandteilen befreit. Das/der Dioxomolybdän(VI)-Salz oder -Komplex kann auch in Salzsäure gelöst werden, das 1,3-Ketoamid eingemischt und die Mischung 10 bis 25 Stunden, vorzugsweise etwa 18 Stunden, bei etwa Raumtemperatur gerührt werden. Das 1,3-Ketoamid kann ferner auch in einem organischen Lösungsmittel, vorzugsweise einem hochsiedenden organischen Lösungsmittel, insbesondere Tetraethylenglykoldimethylether (TEGDME), gelöst zum Einsatz kommen.

Die erfindungsgemäße Dioxomolybdän(VI)-Komplexverbindung kann als Katalysator für härtbare Massen, bevorzugt für Polyurethan-Zusammensetzungen, verwendet werden. Die erfindungsgemäße Dioxomolybdän(VI)-Komplexverbindung beschleunigt die Aushärtung von härtbaren Massen, welche zu Vernetzungsreaktionen fähige Reaktivgruppen aufweisen. Die härtbaren Massen können einkomponentig und mehrkomponentig formuliert sein.

Bevorzugt beschleunigt die erfindungsgemäße Dioxomolybdän(VI)-Komplexverbindung die Aushärtung von zweikomponentigen Polyurethan-Zusammensetzungen, welche mit sich selbst und gegebenenfalls unter dem Einfluss von Feuchtigkeit über blockierte oder insbesondere freie Isocyanatgruppen vernetzen. Dabei wird vor allem die Urethanisierungsreaktion, d.h. die Reaktion von Isocyanatgruppen mit alkoholischen OH-Gruppen, beschleunigt. Die zu vernetzenden Zusammensetzungen können auch weitere zu Vernetzungsreaktionen fähige Reaktivgruppen, wie insbesondere Alkoxysilangruppen, enthalten. Vorzugsweise handelt es sich dabei um Trialkoxysilan-Gruppen, wie sie beispielsweise in Silan-Haftmitteln enthalten sind.

Die erfindungsgemäße Dioxomolybdän(VI)-Komplexverbindung kann vorteilhafterweise als Katalysator in einer zweikomponentigen Polyurethan-Zusammensetzung eingesetzt werden. Diese umfasst neben der erfindungsgemäßen

Dioxomolybdän(VI)-Komplexverbindung ein Polyol als erste Komponente sowie ein Polyisocyanat als zweite Komponente.

Als "zweikomponentig" wird eine Zusammensetzung bezeichnet, bei welcher die Bestandteile derselben in zwei verschiedenen Komponenten vorliegen, welche in voneinander getrennten Gebinden gelagert werden, und welche jeweils für sich lagerstabil sind. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit und/oder erhöhter Temperatur abläuft oder vervollständigt wird.

Mit "Poly" beginnende Substanznamen, wie Polyol oder Polyisocyanat, bezeichnen Substanzen, die formal zwei oder mehr der in ihrem Namen vorkommenden funktionellen Gruppen pro Molekül enthalten.

Der Begriff "Polyisocyanat" umfasst Verbindungen mit zwei oder mehr Isocyanatgruppen, unabhängig davon, ob es sich dabei um monomere Diisocyanate, oligomere Polyisocyanate oder Isocyanatgruppen aufweisende Polymere handelt.

Als Polyisocyanat geeignet ist beispielsweise ein Polyisocyanat in Form eines monomeren Di- oder Triisocyanates oder eines Oligomeren eines monomeren Diisocyanates oder eines Derivates eines monomeren Diisocyanates.

Als monomere Di- oder Triisocyanate geeignet sind beispielsweise 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3-und - 1,4-diisocyanat, l-Methyl-2,4- und -2,6-diisocyanatocyclohexan und beliebige Gemische dieser Isomeren, 1-Isocyanato-3,3,5-trimethyl-5-isocyanatomethyl-cyclohexan (= Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate, wie 3,6-Bis-(9-isocyanatononyl)-4,5-di-(1-heptenyl)-cyclohexen (Dimeryldiisocyanat), $\alpha,\alpha,\alpha',\alpha',\alpha'',\alpha''$-Hexamethyl-1,3,5-mesitylentriisocyanat, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan und Tris-(4-isocyanatophenyl)-thiophosphat.

Bevorzugte Polyisocyanate sind handelsübliche Diisocyanate. Besonders bevorzugt sind HDI, IPDI, TDI und MDI sowie Oligomere von Diisocyanaten und Isocyanatgruppen aufweisende Polyurethanpolymere (NCO-Prepolymere).

Als Polyole können beispielsweise die folgenden handelsüblichen Polyole oder Mischungen davon eingesetzt werden:

- Polyoxyalkylenpolyole, auch Polyetherpolyole oder Oligoetherole genannt, welche Polymerisationsprodukte von Ethylenoxid, 1,2-Propylenoxid, 1,2-oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3-und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Besonders geeignet sind Polyoxyalkylendiole oder Polyoxyalkylentriole, insbesondere Polyoxyethylen- und Polyoxypropylendi- und -triole. Speziell geeignet sind Polyoxyalkylendiole und -triole mit einem Ungesättigtheitsgrad tiefer als 0,02 mEq/g und mit einem Molekulargewicht im Bereich von 1.000 - 30.000 g/mol, sowie Polyoxypropylendiole und -triole mit einem Molekulargewicht von 400 - 8.000 g/mol.

Ebenfalls besonders geeignet sind sogenannte Ethylenoxid-terminierte ("EOendcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.

- Styrol-Acrylnitril- oder Acrylnitril-Methylmethacrylat-gepfropfte Polyetherpolyole.

- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.

Als Polyesterpolyole insbesondere geeignet sind solche, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Capro-lacton und Startern wie den vorgenannten zwei- oder dreiwertigen Alkoholen.

- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.

- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.

- Polyacrylat- und Polymethacrylatpolyole.

- Polyhydroxyfunktionelle Fette und Öle, beispielsweise natürliche Fette und Öle, insbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschließender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschließender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.

- Polykohlenwasserstoffpolyole, auch Oligohydrocarbonole genannt, wie beispielsweise polyhydroxyfunktionelle Polyolefine, Polyisobutylene, Poly-isoprene; polyhydroxyfunktionelle Ethylen-Propylen-, Ethylen-Butylen- oder Ethylen-Propylen-Dien-Copolymere; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylo-nitril/Butadien-Copolymere, wie sie beispielsweise aus Epoxiden oder Aminoalkoholen und carboxylterminierten Acrylonitril/Butadien-Copolymeren hergestellt werden können; sowie hydrierte polyhydroxyfunktionelle Polymere oder Copolymere von Dienen.

Die genannten Polyole weisen bevorzugt ein mittleres Molekulargewicht von 250 - 30.000 g/mol, insbesondere von 400 - 20.000 g/mol, auf und weisen ferner bevorzugt eine mittlere OH-Funktionalität im Bereich von 1,6 bis 3 auf.
Unter "Molekulargewicht" versteht man bei Oligomeren oder Polymeren stets das Molekulargewichtsmittel $M_n$.
Besonders bevorzugt ist der Einsatz von Polyetherpolyolen, vorzugsweise Polypropylenpolyolen und Polyethylen-polypropylen-Mischpolyolen, sowie Polyesterpolyolen und Polycarbonatpolyolen.
Die erfindungsgemäße Dioxomolybdän(VI)-Komplexverbindung liegt vorzugsweise in der ersten Komponente vor, was den Vorteil hat, dass das auf katalytisch wirkende Verbindungen empfindliche Polyisocyanat in der zweiten Komponente in seiner Lagerstabilität (Haltbarkeit) nicht beeinträchtigt wird.
Die erfindungsgemäße Dioxomolybdän(VI)-Komplexverbindung kann als alleiniger Katalysator oder auch zusammen mit anderen Katalysatoren, wie beispielsweise Bismut-, Zinn- oder Zirkoniumverbindungen oder tertiären Aminen, eingesetzt werden.
Die erfindungsgemäße zweikomponentige Polyurethan-Zusammensetzung kann gegebenenfalls weitere üblicherweise eingesetzte Hilfs- und Zusatzstoffe, beispielsweise Pigmente, Weichmacher bzw. Verdünner, Härter, Vernetzer, Kettenverlängerer, weitere Katalysatoren, Haftmittel, Stabilisatoren, Rheologiehilfsmittel und Trocknungsmittel, etc. enthalten.

Die erfindungsgemäße Dioxomolybdän(VI)-Komplexverbindung, betrachtet als Menge elementares Molybdän, liegt in der erfindungsgemäßen zweikomponentigen Polyurethan-Zusammensetzung bevorzugt in einer Menge von 0,002 bis 1 Gew.-%, besonders bevorzugt in einer Menge von 0,01 bis 0,5 Gew.-%, und ganz besonders bevorzugt in einer Menge von 0,02 bis 0,3 Gew.-%, bezogen auf das Gewicht der Zusammensetzung, vor. Zu hohe Mengen führen dazu, dass die Offenzeit bzw. Verarbeitungszeit der Zusammensetzung zu kurz ist, während der Einsatz zu geringer Mengen den Nachteil hat, dass die Zusammensetzung zu schwach katalysiert ist und damit zu langsam, unvollständig und/oder fehlerhaft aushärtet. In der erfindungsgemäßen zweikomponentigen Polyurethan-Zusammensetzung macht die erfindungsgemäße Dioxomolybdän(VI)-Komplexverbindung bevorzugt 0,02 bis 10, bevorzugt 0,1 bis 5, und besonders bevorzugt 0,2 bis 3 mmol-Equivalente Molybdänatome auf 100 g der Zusammensetzung aus.

Wie bereits vorstehend erwähnt, ist die erfindungsgemäße Dioxomolybdän(VI)-Komplexverbindung hinsichtlich der Urethanisierungsreaktion vergleichsweise aktiv sowie auch vergleichsweise selektiv. So zeichnet sich die erfindungsgemäße Dioxomolybdän(VI)-Komplexverbindung sowohl gegenüber Dioxomolybdän(VI)-bis(acetylacetonat) als auch gegenüber Molybdän-carboxylaten durch eine deutlich höhere katalytische Aktivität aus. Die Aushärtung der erfindungsgemäßen zweikomponentigen Polyurethan-Zusammensetzung erfolgt im Allgemeinen rasch. Die Selektivität der erfindungsgemäßen Dioxomolybdän(VI)-Komplexverbindung leidet jedoch nicht unter der erhöhten Aktivität; die Aushärtung erfolgt ohne Bildung von Blasen, selbst bei ungünstigen Bedingungen, wie hoher Temperatur, hoher Umgebungsfeuchte bzw. hohem Restwassergehalt der Zusammensetzung sowie bei Einsatz von Polyolen mit sekundären OH-Gruppen oder hydrophilen Polyolen. Die erfindungsgemäße Dioxomolybdän(VI)-Komplexverbindung ist thermisch und hydrolytisch vergleichsweise stabil, zersetzt sich selbst in einem restwasserhaltigen Polyol nur langsam und behält somit ihre katalytische Aktivität auch bei längerer Lagerdauer. Dennoch führt der Einsatz der erfindungsgemäßen Dioxomolybdän(VI)-Komplexverbindung zu guter Stabilität der ausgehärteten Polyurethan-Zusammensetzung unter thermischer Belastung. Die erfindungsgemäße Dioxomolybdän(VI)-Komplexverbindung ist ferner bei Raumtemperatur flüssig und/oder in Weichmachern oder Polyolen gut löslich und lässt sich somit in bei Raumtemperatur härtenden Systemen auf einfache Weise und insbesondere ohne Verwendung von flüchtigen organischen Lösungsmitteln (VOC) einsetzen. Schließlich ist die erfindungsgemäße Dioxomolybdän(VI)-Komplexverbindung nur schwach farbig und führt kaum zu Verfärbungen der ausgehärteten PolyurethanZusammensetzungen; sie weist auch eine relativ geringe Toxizität auf.

Die erfindungsgemäße zweikomponentige Polyurethan-Zusammensetzung kann in vielen Bereichen eingesetzt werden, beispielsweise als Vergussmasse, Dichtstoff, Klebstoff, Belag, Beschichtung, Lack, Voranstrich, Hartschaum, Weichschaum, Formteil, Elastomer, Faser, Folie oder Membran für Bau- und Industrieanwendungen, beispielsweise als Elektrovergussmasse, Spachtelmasse, Nahtabdichtung, Hohlraumversiegelung, Fugendichtstoff, Montageklebstoff, Karosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag und -beschichtung, Balkon- und Dachbeschichtung, Betonschutzbeschichtung, Parkhausbeschichtung, Rohrbeschichtung, Korrosionsschutzbeschichtung, Textilbeschichtung, Holzlack, Dekorationslack, Primer, Möbelschaum, Polsterschaum, Filterschaum, Isolationsschaum, Schalldämmschaum, Abdichtungsschaum, Verpackungsschaum, Karosserieschaum, Modellbauplatte, Dämpfungselement, Dichtungselement, Reifen, Rollen, Lager, Walze, Förderband, Gummifaden, Schuhsohle, Gehäuse, Fensterprofil, Implantat, Schaumgummi, etc.

Bevorzugte Anwendungsgebiete sind Vergussmassen, Dichtstoffe, Klebstoffe, Beläge, Beschichtungen, Lacke, Voranstriche, Formteile und Elastomere für Bau- und Industrieanwendungen.

Die erfindungsgemäße Dioxomolybdän(VI)-Komplexverbindung kann auch in einkomponentigen Polyurethan-Zusammensetzungen eingesetzt werden. Diese umfassen neben der erfindungsgemäßen Dioxomolybdän(VI)-Komplexverbindung mindestens ein Polyurethanprepolymer mit Isocyanatendgruppen, welches aus mindestens einem Polyisocyanat und mindestens einem Polyol hergestellt ist. Das Polyurethanprepolymer wird in üblicher Weise hergestellt, beispielsweise wie in der EP 1 408 062 A1 beschrieben. Die für die Prepolymerherstellung eingesetzten Polyole sind solche wie in der EP 1 408 062 und vorstehend beschrieben. Gleiches gilt für die zur Herstellung der Polyurethanprepolymere verwendeten Polyisocyanate.

In der erfindungsgemäßen einkomponentigen Polyurethan-Zusammensetzung macht die erfindungsgemäße Dioxomolybdän(VI)-Komplexverbindung 0,01 bis 10, bevorzugt 0,05 bis 2, und besonders bevorzugt 0,1 bis 1 mmol-Equivalente Molybdänatome auf 100 g der Zusammensetzung aus.

Einkomponentige Polyurethanzusammensetzungen enthaltend eine erfindungsgemäße Dioxomolybdän(VI)-Komplexverbindung weisen typischerweise eine vergleichsweise gute Lagerstabilität sowie eine vergleichsweise kurze Hautbildungszeit auf.

Die Anwendungsgebiete der erfindungsgemäßen einkomponentigen Polyurethan-Zusammensetzung entsprechen denjenigen der vorstehend im Zusammenhang mit den zweikomponentigen Polyurethan-Zusammensetzungen erwähnten Anwendungen.

Die erfindungsgemäße Dioxomolybdän(VI)-Komplexverbindung verfügt sowohl über gute thermische sowie auch hydrolytische Stabilität. Sie hydrolysiert daher in einem restwasserhaltigen Polyol nicht schnell und bewahrt so ihre katalytische Aktivität. Ferner ist sie bei Raumtemperatur flüssig und/oder zeigt eine gute Löslichkeit in Weichmachern oder Polyolen. Damit ist ihr Einsatz in bei Raumtemperatur härtenden Systemen auf einfache Weise und ohne Verwendung von flüchtigen organischen Lösungsmitteln (VOC) möglich. Die katalytische Aktivität und Selektivität der erfindungsgemäßen Dioxomolybdän(VI)-Komplexverbindung in Bezug auf die Urethanisierungreaktion ist ausgezeichnet. Mit der erfindungsgemäßen Komplexverbindung ausgehärtete ein- und zweikomponentige Polyurethan-Zusammensetzungen zeichnen sich trotz der Hydrolysestabilität der Komplexverbindung durch gute thermische Beständigkeit aus.

Außer in einkomponentigen und zweikomponentigen Polyurethan-Zusammensetzungen kann die erfindungsgemäße Dioxomolybdän(VI)-Komplexverbindung als Katalysator oder Co-Katalysator auch in anderen härtbaren Massen eingesetzt werden, beispielsweise, in Epoxidharzen, Acrylaten und Silikonen.

**Beispiele**

Beschreibung der Messmethoden

Infrarotspektren wurden auf einem FT-IR Gerät 1600 von Perkin-Elmer gemessen (horizontale ATR-Messeinheit mit ZnSe-Kristall; Messfenster 4000-650 $cm^{-1}$). Flüssige Proben wurden unverdünnt als Filme aufgetragen, feste Proben wurden in $CH_2Cl_2$ gelöst. Die Absorptionsbanden sind in Wellenzahlen ($cm^{-1}$) angegeben.

$^1H$-NMR-Spektren wurden auf einem Spektrometer des Typs Bruker DPX-300 bei 300,13 MHz gemessen; die chemischen Verschiebungen $\delta$ sind angegeben in ppm relativ zu Tetramethylsilan (TMS). Echte und Pseudo-Kopplungsmuster wurden nicht unterschieden.

Die Viskosität wurde auf einem thermostatisierten Kegel-Platten-Viskosimeter Physica MCR 300 (Kegeldurchmesser 20 mm, Kegelwinkel 1°, Kegelspitze-Platten-Abstand 0,05 mm, Schergeschwindigkeit 0,1 bis 100 $s^{-1}$) gemessen.

UV-vis-Spektren von in Dichlormethan gelösten Proben (40 mg/l) in 1 cm Quarzküvetten wurden auf einem Spektrometer des Typs Varian Cary 50 im Wellenlängenbereich 800 - 200 nm gemessen. Angegeben sind die Extinktionsmaxima $\lambda_{max}$ in nm und in Klammern die zugehörigen Extinktionskoeffizienten $\varepsilon$ in $l \cdot g^{-1} \cdot cm^{-1}$.

Herstellung der Dioxomolybdän(VI)-Komplexverbindungen

**Allgemeine Herstellvorschrift A**

In einem Rundkolben wurden Dioxomolybdän(VI)-bis(acetylacetonat) und ein 1,3-Ketoamid vermischt und die Mischung unter Rühren während 2 Stunden auf 80 °C erwärmt. Darauf wurde das Reaktionsgemisch im Vakuum von den flüchtigen Bestandteilen befreit.

**Allgemeine Herstellvorschrift B**

In einem Rundkolben wurde Natriummolybdat-dihydrat ($Na_2MoO_4 \cdot 2H_2O$) in 15 ml SalzsäureLösung (0,1 M) gelöst. In diese Lösung wurde ein 1,3-Ketoamid eingemischt und die Mischung während 18 Stunden bei 23 °C gerührt. Darauf wurde das Reaktionsgemisch filtriert und der erhaltene Feststoff im Vakuum getrocknet.

**Allgemeine Herstellvorschrift C**

In einem Rundkolben wurde eine Mischung von Dioxomolybdän(VI)-bis(acetylacetonat) und einem 1,3-Ketoamid in Tetraethylenglykoldimethylether (TEGDME) unter Rühren während 3 Stunden auf 80 °C erwärmt. Darauf wurde das Reaktionsgemisch auf Raumtemperatur gekühlt.

**Beispiel 1: Dioxomolybdän(VI)-bis(N,N-diethyl-3-oxobutanamidat)**

Gemäß der allgemeinen Herstellvorschrift A wurden 3,26 g Dioxomolybdän(VI)-bis(acetylacetonat) und 3,33 g N,N-Diethyl-3-oxobutanamid umgesetzt. Man erhielt 4,49 g eines gelben, mikrokristallinen Festkörpers.
$^1H$-NMR ($CDCl_3$): $\delta$ 1,5 und 1,9 (2×*t*, 12 H, $CH_3CH_2$), 2,0 (*s*, 6 H, $CH_3CO$), 3,15, 3,3 und 3,55 (3×*dq*, 8 H, $CH_3CH_2$), 5,2 (*s*, 2 H, enol-CH).
FT-IR: 2974, 2932, 2872, 1720, 1594, 1491, 1437, 1358, 1308, 1276, 1197, 1081, 1015, 961, 929, 903, 773, 660.
UV-vis: 309 (0,11) und 275 (0,23). (vgl. Dioxomolybdän(VI)-bis(acetylacetonat): 314 (0,13) und 270 (0,27).)

**Beispiel 2: Dioxomolybdän(VI)-bis(N,N-diethyl-3-oxobutanamidat) in TEGDME**

Gemäß der allgemeinen Herstellvorschrift C wurden 6,54 g Dioxomolybdän(VI)-bis(acetylacetonat) und 7,21 g N,N-Diethyl-3-oxobutanamid in 18,00 g TEGDME umgesetzt. Man erhielt 31,75 g einer orangefarbenen Lösung.

**Beispiel 3: Dioxomolybdän(VI)-bis(N,N-dibutyl-3-oxobutanamidat)**

Gemäß der allgemeinen Herstellvorschrift A wurden 3,26 g Dioxomolybdän(VI)-bis(acetylacetonat) und 4,48 g N,N-Dibutyl-3-oxobutanamid umgesetzt. Man erhielt 6,12 g eines gelben Festkörpers.
$^1$H-NMR ($CDCl_3$): δ 0,9 (*dt,* 12 H, Me), 1,2-1,5 (*m,* 8 H, C$H_2$Me), 1,5-1,6 (*m,* 8 H, C$H_2CH_2$N), 2,0 (*s*, 6 H, MeCO), 2,9-3,0 (*m*, 4 H, $NCH_2$), 3,1-3,3 (*m*, 8 H, $NCH_2$), 3,5-3,65 (*m*, 4 H, $NCH_2$), 5,15 (*s*, 2 H, enol-CH).
FT-IR: 3500, 2916, 2854, 1717, 1582, 1505, 1445, 1361, 1255, 1192, 1112, 987, 958, 904, 860, 771, 731, 670.

**Beispiel 4: Dioxomolybdän(VI)-bis(N,N-bis(2-methoxyethyl)-3-oxobutanamidat)**

Gemäß der allgemeinen Herstellvorschrift A wurden 3,26 g Dioxomolybdän(VI)-bis(acetylacetonat) und 4,56 g N,N-Bis(2-methoxyethyl)-3-oxobutanamid umgesetzt. Man erhielt 6,15 g eines bräunlichen, hochviskosen Öls.
$^1$H-NMR ($CDCl_3$): δ 1,95, 2,0, 2,05, 2,15 (4x*s*, 6 H, MeCO), 3,35 (*s*, 12 H, OMe), 3,4-3,8 (*m*, 16 H, $NCH_2$ und $OCH_2$), 5,3, 5,4, 5,5 und 5,7 (4x*s*, 2 H, enol-CH)
FT-IR: 2925, 2889, 1718, 1636, 1587, 1501, 1430, 1355, 1274, 1191, 1110, 1007, 960, 927, 898, 774, 662.

**Beispiel 5: Dioxomolybdän(VI)-bis(N,N-bis(2-methoxyethyl)-3-oxobutanamidat) in TEGDME**

Gemäß der allgemeinen Herstellvorschrift C wurden 3,26 g Dioxomolybdän(VI)-bis(acetylacetonat) und 4,79 g N,N-Bis(2-methoxyethyl)-3-oxobutanamid in 9,16 g TEGDME umgesetzt. Man erhielt 17,21 g einer orangefarbenen Lösung.

**Beispiel 6: Dioxomolybdän(VI)-bis(N-cyclohexyl-N-methyl-3-oxobutanamidat)**

Gemäß der allgemeinen Herstellvorschrift A wurden 3,26 g Dioxomolybdän(VI)-bis(acetylacetonat) und 4,14 g N-Cyclohexyl-N-methyl-3-oxobutanamid umgesetzt. Man erhielt 5,94 g eines orangefarbenen, hochviskosen Öls.
$^1$H-NMR ($CDCl_3$): δ 1,0-1,9 (*m*, 20 H, $CH_2$), 2,0-2,1 (*m*, 6 H, MeCO), 2,3 (*s*, 3H, Me vom Ligand), 2,75-2,9 (4×*s*, 6 H, NMe), 4,2-4,5 (*m*, 2 H, CHN), 5,15-5,6 (8×*s*, 2 H, enol-CH).
FT-IR: 2926, 2854, 1719, 1601, 1508, 1449, 1346, 1316, 1265, 1199, 1164, 1010, 956, 932, 902, 773, 731, 701.

**Beispiel 7: Dioxomolybdän(VI)-bis(N-cyclohexyl-N-methyl-3-oxobutanamidat) in TEGDME**

Gemäß der allgemeinen Herstellvorschrift C wurden 3,31 g Dioxomolybdän(VI)-bis(acetylacetonat) und 4,49 g N-Cyclohexyl-N-methyl-3-oxobutanamid in 10,50 g TEGDME umgesetzt. Man erhielt 18,30 g einer orangefarbenen Lösung.

**Beispiel 8: Dioxomolybdän(VI)-bis(1-morpholinobutan-1,3-dionat)**

Gemäß der allgemeinen Herstellvorschrift A wurden 3,26 g Dioxomolybdän(VI)-bis(acetylacetonat) und 3,59 g 1-Morpholinobutan-1,3-dion umgesetzt. Man erhielt 5,41 g eines gelben Festkörpers.
$^1$H-NMR ($CDCl_3$): δ 2,05 (*s*, 3 H, MeCO), 3,4-3,8 (*m*, 16 H, $NCH_2$ und $OCH_2$), 5,25 (*s*, 2 H, enol-CH).
FT-IR: 2955, 2870, 1734, 1606, 1505, 1464, 1433, 1366, 1293, 1228, 1193, 955, 931, 901, 772, 734, 697.

**Beispiel 9: Dioxomolybdän(VI)-bis(1-(4-methylpiperazin-1-yl)butan-1,3-dionat)**

Gemäß der allgemeinen Herstellvorschrift A wurden 3,26 g Dioxomolybdän(VI)-bis(acetylacetonat) und 3,87 g 1-(4-Methylpiperazin-1-yl)butan-1,3-dion umgesetzt. Man erhielt 6,02 g eines orangefarbenen, hochviskosen Öls.
$^1$H-NMR ($CDCl_3$): δ 2,05 (*s*, 6 H, MeCO), 2,3 (d, 6 H, NMe), 2,4 (*dd,* 8 H, $NCH_2$), 3,45 (*dd,* 4H, $CH_2$N), 3,65 (*dd,* 2H, $CH_2$N), 5,15, 5,25, 5,5 (3×*s*, 2H, enol-CH).
FT-IR: 3411, 2938, 2791, 1719, 1606, 1499, 1446, 1360, 1291, 1260, 1142, 991, 959, 930, 903, 772, 669.

**Beispiel 10: Dioxomolybdän(VI)-bis(N,N-dibutyl-3-oxoheptanamidat)**

Gemäß der allgemeinen Herstellvorschrift A wurden 3,19 g Dioxomolybdän(VI)-bis(acetylacetonat) und 5,26 g N,N-Dibutyl-3-oxoheptanamid umgesetzt. Man erhielt 6,52 g eines orange-gelben, hochviskosen Öls.
$^1$H-NMR ($CDCl_3$): δ 0,85-1,0 (*m*, 18 H, Me), 1,3-1,45 (*m*, 12 H, C*H*$_2$Me), 1,5-1,65 (*m*, 12 H, C*H*$_2$CH$_2$Me), 2,05-2,3 (*m*, 4 H, $CH_2CO$), 2,9-3,0 (*m,* 2 H, $CH_2N$), 3,15-3,45 (*m,* 6 H, $CH_2N$), 3,5-3,65 (br *s*, 2 H, übrige $CH_2$), 5,15, 5,25 und 5,7 (3x*s*, 2 H, enol-CH).
FT-IR: 2954, 2870, 1737, 1604, 1584, 1501, 1463, 1369, 1292, 1226, 1185, 930, 902, 774, 733.

**Beispiel 11: Dioxomolybdän(VI)-bis(N,N-dibutyl-3-oxoheptanamidat) in TEGDME**

Gemäß der allgemeinen Herstellvorschrift C wurden 3,24 g Dioxomolybdän(VI)-bis(acetylacetonat) und 5,58 g N,N-Dibutyl-3-oxoheptanamid in 6,70 g TEGDME umgesetzt. Man erhielt 15,52 g einer orangefarbenen Lösung.

**Beispiel 12: Dioxomolybdän(VI)-bis(N,N-bis(2-methoxyethyl)-3-oxoheptanamidat)**

Gemäß der allgemeinen Herstellvorschrift A wurden 3,72 g Dioxomolybdän(VI)-bis(acetylacetonat) und 6,21 g N,N-Bis(2-methoxyethyl)-3-oxoheptanamid umgesetzt. Man erhielt 7,66 g eines orange-gelben Festkörpers.
$^1$H-NMR ($CDCl_3$): δ 0,9 (*t*, 6 H, Me), 1,35 (*dq,* 4 H, C*H*$_2$Me), 1,5-1,7 (*m*, 4 H, C*H*$_2$CH$_2$Me), 2,25 (*t,* 4 H, $CH_2CO$), 3,35 (*s*, 12 H, OMe), 3,3-3,8 (*m*, 20 H, $CH_2O$, $CH_2N$ und $CH_2$), 5,3 (*s*, 2 H, enol-CH).
FT-IR: 2927, 2871, 1746, 1716, 1602, 1502, 1372, 1274, 1185, 1115, 1012, 957, 927, 904, 775.

**Beispiel 13: Dioxomolybdän(VI)-bis(N-cyclohexyl-N-methyl-3-oxoheptanamidat)**

Gemäß der allgemeinen Herstellvorschrift A wurden 3,66 g Dioxomolybdän(VI)-bis(acetylacetonat) und 5,03 g N-Cyclohexyl-N-methyl-3-oxoheptanamid umgesetzt. Man erhielt 6,86 g eines bräunlichen, hochviskosen Öls.
$^1$H-NMR (DMSO-$d_6$): δ 0,7-0,9 (*m*, 6 H, Me), 1,0-1,9 (*m*, 28 H, $CH_2$Me, C*H*$_2$CH$_2$Me, $CH_2^{cy}$), 2,2-2,3 (*m*, 4 H, $CH_2CO$), 2,7-2,9 (*m*, 6 H, NMe), 4,2-4,3 (*m*, 2 H, CHN), 5,4, 5,5, 5,7, 5,9 (4xs, 2 H, enol-CH).
FT-IR: 2926, 2855, 1739, 1717, 1601, 1498, 1449, 1350, 1318, 1254, 1189, 1164, 1025, 970, 930, 900, 774, 729, 667.

**Beispiel 14: Dioxomolybdän(VI)-bis(1-morpholinoheptan-1,3-dionat)**

Gemäß der allgemeinen Herstellvorschrift A wurden 3,66 g Dioxomolybdän(VI)-bis(acetylacetonat) und 5,03 g 1-Morpholinoheptan-1,3-dion umgesetzt. Man erhielt 6,58 g eines bräunlichen, hochviskosen Öls.
$^1$H-NMR (DMSO-$d_6$): δ 0,8-0,9 (*m*, 6 H, Me), 1,2-1,35 (*m*, 4 H, C*H*$_2$Me), 1,4-1,5 (*m*, 4 H, C*H*$_2$CH$_2$Me), 3,3-3,7 (*m*, 16 H, $CH_2O$, $CH_2N$ und $CH_2$), 5,55, 5,65, 5,95 (3xs, 2 H, enol-CH).
FT-IR: 2956, 2857, 1717, 1600, 1500, 1441, 1370, 1251, 1185, 1113, 929, 900, 861, 772, 667.

**Beispiel 15: Dioxomolybdän(VI)-bis(N,N-diethyl-3-phenyl-3-oxopropanamidat)**

Gemäß der allgemeinen Herstellvorschrift B wurden 2,05 g Natriummolybat-dihydrat und 4,33 g N,N-Diethyl-3-phenyl-3-oxopropanamid umgesetzt. Man erhielt 5,18 g eines gelben, mikrokristallinen Festkörpers.
$^1$H-NMR (DMSO-$d_6$): δ 0,9 und 1,15 (2×*t*, 12 H, Me), 3,1-3,6 (*m*, 8 H, C*H*$_2$Me), 6,1 (*s*, 2 H, enol-CH), 7,45-7,5 (*m*, 6 H, arom-H), 7,8-7,85 (*m*, 4 H, arom-H).
FT-IR: 2975, 1605, 1573, 488, 1439, 1357, 1282, 1238, 1101, 929, 899, 765, 694, 673.

**Beispiel 16: Dioxomolybdän(VI)-bis(N,N-dibutyl-3-oxo-3-phenylpropanamidat)**

Gemäß der allgemeinen Herstellvorschrift A wurden 3,26 g Dioxomolybdän(VI)-bis(acetylacetonat) und 5,78 g N,N-Dibutyl-3-oxo-3-phenylpropanamid umgesetzt. Man erhielt 7,35 g eines gelben Festkörpers.
$^1$H-NMR ($CDCl_3$): δ 0,7 (*t*, 12 H, Me), 0,9-1,0 (*m,* 12 H, Me), 1,3-1,45 (*m,* 8 H, C*H*$_2$Me), 1,5-1,7 (*m,* 8 H, C*H*$_2$CH2Me), 2,9-3,05 (*m,* 2 H, $CH_2N$), 3,2-3,4 (*m,* 2 H, $CH_2N$), 3,45-3,6 (*m,* 2 H, $CH_2N$), 5,7, 5,9, 5,95 (4x*s*, 2H, enol-CH), 7,35-7,45 (*m*, 6 H, arom-H), 7,75-7,8 (*m*, 4 H, arom-H).
FT-IR: 2955, 2869, 1740, 1684, 1604, 1572, 1488, 1358, 1294, 1215, 1104, 1022, 932, 901, 765, 734, 692.

**Beispiel 17: Dioxomolybdän(VI)-bis(N,N-dibutyl-3-oxo-3-phenylpropanamidat) in TEGDME**

Gemäß der allgemeinen Herstellvorschrift C wurden 3,37 g Dioxomolybdän(VI)-bis(acetylacetonat) und 6,25

g N,N-Dibutyl-3-oxo-3-phenylpropanamid in 6,76 g TEGDME umgesetzt. Man erhielt 16,38 g einer gelborangen Lösung.

**Beispiel 18: Dioxomolybdän(VI)-bis(1-morpholino-3-phenylpropan-1,3-dionat)**

Gemäß der allgemeinen Herstellvorschrift A wurden 3,26 g Dioxomolybdän(VI)-bis(acetylacetonat) und 4,90 g 1-Morpholino-3-phenylpropan-1,3-dion umgesetzt. Man erhielt 7,09 g eines orange-gelben Festkörpers.
$^1$H-NMR ($CDCl_3$): δ 3,4-3,85 (*m*, 16 H, $CH_2N$ und $CH_2O$), 5,5, 5,75, 5,8, 5,9 und 5,05 (5xs, 2 H, enol-CH), 7,35-7,5 *(m,* 6 H, arom-H), 7,75-7,85 *(m,* 2 H, arom-H), 8,0-8,1 *(m,* 2 H, arom-H).
FT-IR: 3056, 2967, 2918, 2856, 2359, 1684, 1571, 1496, 1358, 1263, 1234, 1114, 1051, 1026, 933, 901, 763, 731, 689.

**Beispiel 19: Dioxomolybdän(VI)-bis(N,N-dibutyl-2-oxocyclopentancarboxamidat)**

Gemäß der allgemeinen Herstellvorschrift A wurden 3,26 g Dioxomolybdän(VI)-bis(acetylacetonat) und 5,02 g N,N-Dibutyl-2-oxocyclopentancarboxamid umgesetzt. Man erhielt 7,90 g eines gelben Festkörpers.
$^1$H-NMR ($CDCl_3$): δ 0,9-1,0 (2×*t*, 6 H, $CH_2CH_3$), 1,25-1,4 (*m,* 8 H, $CH_2CH_3$), 1,4-1,6 (*m,* 8 H, $CH_2CH_2CH_3$), 1,8-1,95 (*m*, 2 H, $CH_2^{cy}$), 2,15-2,55 (*m*, 10 H, $CH_2^{cy}$), 3,1-3,2 (*m*, 4 H, $NCH_2$), 3,35 (*t*, 1 H, CHCO), 3,45-3,6 (*m*, 4 H, $NCH_2$), 5,5, 5,7 und 5,8 (3xs, 2 H, enol-CH).
FT-IR: 2955, 2871, 1739, 1632, 1587, 1562, 1516, 1456, 1371, 1265, 1230, 1104, 1026, 932, 903, 796, 734, 699, 668.

**Beispiel 20: Dioxomolybdän(VI)-bis(N,N-bis-dibutyl-2-oxocyclopentancarbox-amidat) in TEGDME**

Gemäß der allgemeinen Herstellvorschrift C wurden 3,29 g Dioxomolybdän(VI)-bis(acetylacetonat) und 5,10 g N,N-Dibutyl-2-oxocyclopentancarboxamid in 8,78 g TEGDME umgesetzt. Man erhielt 17,17 g einer orangebraunen Lösung, in der sich beim Stehen Kristalle bildeten.

**Beispiel 21: Dioxomolybdän(VI)-acetylacetonat-(N,N-diethyl-3-oxobutanamidat)**

Gemäß der allgemeinen Herstellvorschrift A wurden 4,24 g Dioxomolybdän(VI)-bis(acetylacetonat) und 2,12 g N,N-Diethyl-3-oxobutanamid umgesetzt. Man erhielt 4,95 g eines gelben, mikrokristallinen Festkörpers.
$^1$H-NMR (DMSO-$d_6$): δ 0,9 und 1,1 (2×*t*, 12 H, Me), 1,9, 2,0 und 2,15 (3x*s*, 9 H, MeCO), 3,1-3,4 (*m*, 4 H, $CH_2N$), 5,45-6,0 (6×*s*, 2 H, enol-CH).
FT-IR: 2976, 2934, 1715, 1588, 1504, 1437, 1357, 1308, 1265, 1197, 1080, 1017, 963, 931, 904, 783, 666.

**Beispiel 22: Dioxomolybdän(VI)-(N,N-diethyl-3-oxobutanamidat)-(N,N-bis(2-methoxyethyl)-3-oxobutanamidat))**

Gemäß der allgemeinen Herstellvorschrift A wurden 3,35 g Dioxomolybdän(VI)-bis(acetylacetonat) und eine Kombination aus 1,71 g N,N-Diethyl-3-oxobutanamid und 2,35 g N,N-Bis(2-methoxyethyl)-3-oxobutanamid umgesetzt. Man erhielt 5,42 g eines rötlichen, hochviskosen Öls.
$^1$H-NMR (DMSO-$d_6$): δ 0,95-1,0 (*dt,* 6 H, Me), 1,05-1,15 (*m*, 6 H, Me), 1,9-2,5 (3×*s*, 6 H, Me-CO), 3,2-3,6 (*m*, 12 H, $CH_2N$ und $CH_2O$), 5,5 (*dd,* 2 H, enol-CH), 5,9 (*d*, 1 H, enol-CH).
FT-IR: 2980, 2889, 1718, 1587, 1499, 1453, 1436, 1355, 1305, 1275, 1195, 1112, 1013, 960, 925, 897, 773, 658.

<u>Einkomponentige Polyurethan-Zusammensetzungen</u>

**Beispiele 23 bis 26 und Vergleichsbeispiele V1 bis V2:**

In einem Polypropylenbecher mit Schraubverschluss wurde für jedes Beispiel das Polyurethanpolymer ***P1***, dessen Herstellung nachfolgend beschrieben wird, mittels eines Zentrifugalmischers (SpeedMixer™ DAC 150, FlackTek Inc.; 1 min. bei 2500 U/min) mit einem Katalysator zu einer homogenen Masse vermischt und die so erhaltene Masse sofort in eine innenlackierte Aluminiumtube abgefüllt und diese luftdicht verschlossen.
Das Polyurethanpolymer ***P1*** wurde wie folgt hergestellt:
1300 g Polyoxypropylen-Diol (Acclaim® 4200 N, von Bayer; OH-Zahl 28,5 mg KOH/g), 2600 g Polyoxypropylenpolyoxyethylen-Triol (Caradol® MD34-02, von Shell; OH-Zahl 35,0 mg KOH/g), 600 g 4,4'-Methylendiphenyldiisocyanat (4,4'-MDI; Desmodur® 44 MC L, von Bayer) und 500 g Diisodecylphthalat (DIDP; Palatinol® Z, von BASF) wurden nach bekanntem Verfahren bei 80 °C zu einem NCO-terminierten Polyurethanpolymeren mit einem Gehalt an freien IsocyanatGruppen von 2,05 Gewichts-% umgesetzt.
Die so erhaltenen Zusammensetzungen wurden auf Lagerstabilität sowie auf Aushärtungsgeschwindigkeit geprüft.

Als Maß für die Lagerstabilität wurde die Veränderung der Viskosität während der Lagerung in der Wärme bestimmt. Dazu wurden die Zusammensetzungen in der verschlossenen Tube im Ofen bei 60 °C gelagert und die Viskosität bei 20 °C ein erstes Mal nach 4 Stunden (= "Viskosität frisch") und ein zweites Mal nach 7 Tagen (= "Viskosität gelagert") Lagerdauer gemessen. Die Lagerstabilität ergibt sich aus der prozentualen Zunahme des zweiten Viskositätswerts gegenüber dem ersten. Dazu wird die Viskositätszunahme in % gemäß folgender Formel berechnet:

rechnet:

$$[(\text{Viskosität nach 7 d} / \text{Viskosität nach 4 h}) - 1] \times 100\%.$$

Als Maß für die Aushärtungsgeschwindigkeit wurde die Zeit bis zur Klebefreiheit (Hautbildungszeit) bestimmt, und zwar für die während 4 Stunden bei 60 °C gelagerten Zusammensetzungen (= "HBZ frisch") und für die während 7 Tagen bei 60 °C gelagerten Zusammensetzungen (= "HBZ gelagert"). Dazu wurden die raumtemperaturwarmen Zusammensetzungen in einer Schichtdicke von ca. 3 mm auf Pappkarton aufgetragen und im Normklima ("NK"; 23±1 °C, 50±5% relative Luftfeuchtigkeit) jeweils die Zeit bestimmt, die es dauerte, bis beim leichten Antippen der Oberfläche einer Zusammensetzung mittels einer Pipette aus LDPE erstmals keine Rückstände auf der Pipette mehr zurückblieben.

Die Ergebnisse dieser Prüfungen sind in der **Tabelle 1** aufgeführt.

| Beispiel | **23** | **24** | **25** | **26** | **V1** | **V2** |
|---|---|---|---|---|---|---|
| Polyurethanpolymer ***P1*** | 50 | 50 | 50 | 50 | 50 | 50 |
| Katalysator **Beispiel 2** | 0,72 | - | - | - | - | - |
| Katalysator **Beispiel 5** | - | 0,78 | - | - | - | - |
| Katalysator **Beispiel 10** | - | - | 0,30 | - | - | - |
| Katalysator **Beispiel 20** | - | - | - | 0,77 | - | - |
| $MoO_2(acac)_2$[a] | - | - | - | - | 0,73 | - |
| Molybdäncarboxylat[b] | - | - | - | - | - | 0,29 |
| mmol-Equiv./100g[c] | 0,9 | 0,9 | 0,9 | 0,9 | 0,9 | 0,9 |
| Viskosität frisch (Pa·s) | 57,7 | 57,3 | 81,6 | 59,7 | 122 | 64,7 |
| Viskosität gelagert (Pa·s) | 98,0 | 87,1 | 111 | 104 | geliert | 84,6 |
| Viskositätszunahme (%) | 70 | 52 | 36 | 74 | >300 | 31 |
| HBZ frisch (min.) | 86 | 94 | 145 | 95 | 83 | >360 |
| HBZ gelagert (min.) | 150 | 154 | 170 | 110 | - | >360 |

**Tabelle 1:** **Einkomponentige Polyurethan-Zusammensetzungen** (Mengen in Gewichtsteilen).
[a] 20%ige Lösung in Methylethylketon. [b] Molybdän-2-ethylhexanoat (15% Mo, von Shepherd). [c] mmol-Equivalente Molybdänatome des Katalysators auf 100 g der Zusammensetzung.

Aus der Tabelle 1 ist ersichtlich, dass die einkomponentigen Polyurethanzusammensetzungen mit den erfindungsgemäßen Katalysatoren vergleichsweise gute Lagerstabilitäten und Hautbildungszeiten aufweisen.

Zweikomponentige Polyurethan-Zusammensetzungen

**Beispiele 27 bis 28 und Vergleichsbeispiele V3 bis V7**

Zur Herstellung der ersten Komponente wurden für jedes Beispiel ein Polyethertriol (Voranol® CP 4755, von Dow) und ein Katalysator gemäß Tabelle 2 in einem Zentrifugalmischer (Speed-Mixer™ DAC 150, FlackTek Inc.) während 30 sec. bei 3000 U/min. innig vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die beschriebene Weise mit einem modifizierten, bei Raumtemperatur flüssigen Diphenylmethandiisocyanat (Desmodur® CD-L, von Bayer) als zweite Komponente gemäß Tabelle 2 zu einer Polyurethan-Zusammensetzung vermischt.

Ebenso wurde für jedes Beispiel die während 7 Tagen bei 60 °C gelagerte erste Komponente mit der zweiten Komponente gemäß **Tabelle 2** auf die gleiche Weise zu einer Polyurethan-Zusammensetzung vermischt.

| Beispiel | **27** | **28** | **V3** | **V4** | **V5** | **V6** | **V7** |
|---|---|---|---|---|---|---|---|
| *Erste Komponente:* | | | | | | | |
| Voranol® CP 4755 | 50 | 50 | 50 | 50 | 50 | 50 | 50 |
| Katalysator **Beispiel 2** | 0,71 | - | - | - | - | - | - |
| Katalysator **Beispiel 11** | - | 0,75 | - | - | - | - | - |
| $MoO_2(acac)_2$[a] | - | - | 1,74 | - | - | - | - |
| Molybdäncarboxylat[b] | - | - | - | 0,81 | - | - | - |
| DBTDL[c] | - | - | - | - | 0,46 | - | - |
| Coscat® 83[d] | - | - | - | - | - | 0,02 | - |
| DABCO 33-LV®[e] | - | - | - | - | - | - | 0,10 |
| mmol-Equiv./100g[f] | 0,80 | 0,86 | 2,68 | 2,27 | 0,13 | 0,03 | 1,07 |
| *Zweite Komponente:* | | | | | | | |
| Desmodur® CD-L | 5,10 | 5,10 | 5,10 | 5,10 | 5,10 | 5,10 | 5,10 |

**Tabelle 2:** **Zweikomponentige Polyurethan-Zusammensetzungen** (Mengen in Gewichtsteilen).
[a] 28,6%ige Suspension in Methylethylketon. [b] Molybdän-2-ethylhexan-oat (15% Mo, von Shepherd). [c] 10%ige Lösung von Dibutylzinn-dilaurat in Diisodecylphthalat. [d] Bismut-tris(neodecanoat) in Neodecansäure (16% Bi, von Erbslöh). [e] 33%ige Lösung von 1,4-Diazabicyclo[2.2.2]-octan in Dipropylenglykol (von Air Products). [f] mmol-Equivalente Metallatome bzw. Aminogruppen des Katalysators auf 100 g der Zusammensetzung.

Die Polyurethan-Zusammensetzungen wurden auf Aspekt, Zeit bis zur Klebefreiheit, Blasenbildung sowie Shore A-Härte geprüft, und zwar jeweils sowohl für die Zusammensetzung mit der frisch hergestellten ersten Komponente als auch für die Zusammensetzung mit der während 7 Tagen bei 60 °C gelagerten ersten Komponente. Ausschließlich für die Zusammensetzung mit der frisch hergestellten ersten Komponente wurde weiterhin die mechanischen Eigenschaften im Zugversuch gemessen, und zwar vor und nach verschiedenen Lagerungen zur beschleunigten Alterung der Proben. Der Aspekt der Zusammensetzung wurde rein optisch beurteilt und als "klar", "trüb" oder inhomogen ("inh.") gewertet.

Die Zeit bis zur Klebefreiheit (Hautbildungszeit) wurde wie für Beispiel 23 beschrieben gemessen.

Die Blasenbildung wurde optisch beurteilt anhand der Menge ("viele", "einige", "keine") an Gasblasen, die in der für die Bestimmung der Hautbildungszeit angesetzten Zusammensetzung während deren Aushärtung auftrat.

Die Shore A-Härte wurde bestimmt nach DIN 53505 an während 7 Tagen im Normklima ausgehärteten Prüfkörpern.

Zur Bestimmung der mechanischen Eigenschaften im Zugversuch wurde von den Zusammensetzungen Filme von ca. 3 mm Dicke hergestellt, indem die Zusammensetzung in eine plane PTFE-Form gegossen und während 7 Tagen im Normklima ausgehärtet wurde. Es wurden klebfreie und elastische Filme erhalten. Aus den Filmen wurden Hanteln mit einer Länge von 75 mm, bei einer Steglänge von 30 mm und einer Stegbreite von 4 mm, ausgestanzt und ein Teil davon gemäß DIN EN 53504 bei einer Zuggeschwindigkeit von 200 mm/min auf Zugfestigkeit, Bruchdehnung und E-Modul (bei 0,5 bis 5,0% Dehnung) geprüft. Der verbleibende Teil der Hanteln wurde während 1 Tag bei 100 °C im Umluftofen, bzw. während 10 Tagen unter "Kataplasma" (40°C und 100% relative Luftfeuchtigkeit), bzw. während 10 Tagen unter "Kataplasma" sowie 1 Tag bei 100 °C, gelagert, darauf jeweils während einem Tag im Normklima gehalten und gemäß DIN EN 53504 wie beschrieben geprüft.

Die Ergebnisse dieser Prüfungen sind in der **Tabelle 3** aufgeführt.

| Beispiel | 27 | 28 | V3 | V4 | V5 | V6 | V7 |
|---|---|---|---|---|---|---|---|
| *Zusammensetzung mit frisch hergestellter erster Komponente:* | | | | | | | |
| Aspekt | klar | klar | inh. | klar | klar | klar | klar |
| Hautbildungszeit (min.) | 9 | 15 | 35 | 110 | 10 | 3 | 15 |
| Shore A-Härte | 42 | 42 | 16 | 25 | 48 | 44 | 33 |
| Blasenbildung | keine | keine | keine | einige | einige | keine | einige |
| Zugfestigkeit (MPa): 7d/NK | 0,86 | 0,76 | 0,54 | 0,66 | 0,76 | 0,54 | 0,90 |
| +10d/Kataplasma | 0,75 | 0,80 | 0,62 | 0,71 | 0,71 | 0,79 | 0,82 |
| +1d/100°C | 0,92 | 0,82 | 0,66 | 0,68 | 0,60 | 0,73 | 0,86 |
| +10d/Kataplasma+1d/100°C | 0,85 | 0,86 | 0,61 | 0,77 | 0,65 | 0,73 | 0,89 |
| Bruchdehnung (%): 7d/NK | 78 | 67 | 81 | 83 | 65 | 42 | 100 |
| +10d/Kataplasma | 63 | 72 | 105 | 85 | 56 | 73 | 85 |
| +1d/100°C | 90 | 91 | 123 | 108 | 168 | 72 | 105 |
| +10d/Kataplasma+1d/100°C | 83 | 97 | 110 | 124 | 170 | 74 | 108 |
| E-Modul (MPa): 7d/NK | 1,66 | 1,58 | 0,85 | 1,21 | 1,68 | 1,46 | 1,44 |
| +10d/Kataplasma | 1,66 | 1,57 | 0,85 | 1,21 | 1,68 | 1,56 | 1,47 |
| +1d/100°C | 1,57 | 1,36 | 0,92 | 1,00 | 0,60 | 1,49 | 1,23 |
| +10d/Kataplasma+1d/100°C | 1,50 | 1,36 | 0,80 | 1,06 | 0,71 | 1,41 | 1,23 |
| *Zusammensetzung mit gelagerter* | | | | | | | |
| Aspekt | klar | klar | inh. | klar | klar | klar | klar |
| Hautbildungszeit (min.) | 9 | 15 | 30 | 120 | 10 | 45 | 15 |
| Shore A-Härte | 44 | 46 | 32 | 34 | 48 | 45 | 32 |
| Blasenbildung | keine | keine | keine | einige | einige | einige | einige |
| **Tabelle 3: Eigenschaften der zweikomp. Polyurethan-Zusammensetzungen** | | | | | | | |

Aus der Tabelle 3 ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und blasenfrei zu einem Material mit vergleichsweise hoher Festigkeit und guter Beständigkeit aushärten.

Beispiele 29 bis 30 und Vergleichsbeispiele V8 bis V12

Zur Herstellung der ersten Komponente wurden für jedes Beispiel ein Polyethertriol (Voranol® CP 4755, von Dow), ein Polyetherdiol (Acclaim® 4200, von Bayer) und ein Katalysator gemä-Tabelle 4 in einem Zentrifugalmischer (SpeedMixer™ DAC 150, FlackTek Inc.) während 30 sec. bei 3000 U/min. innig vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die beschriebene Weise mit einem modifizierten, bei Raumtemperatur flüssigen Diphenylmethandiisocyanat (Desmodur® CD-L, von Bayer) als zweite Komponente gemäß Tabelle 4 zu einer Polyurethan-Zusammensetzung vermischt.

Ebenso wurde für jedes Beispiel die während 7 Tagen bei 60 °C gelagerte erste Komponente mit der zweiten Komponente gemäß **Tabelle 4** auf die gleiche Weise zu einer Polyurethan-Zusammensetzung vermischt.

| Beispiel | 29 | 30 | V8 | V9 | V10 | V11 | V12 |
|---|---|---|---|---|---|---|---|
| *Erste Komponente:* | | | | | | | |
| Voranol® CP 4755 | 33,3 | 33,3 | 33,3 | 33,3 | 33,3 | 33,3 | 33,3 |
| Acclaim® 4200 | 16,7 | 16,7 | 16,7 | 16,7 | 16,7 | 16,7 | 16,7 |

(fortgesetzt)

| Beispiel | 29 | 30 | V8 | V9 | V10 | V11 | V12 |
|---|---|---|---|---|---|---|---|
| *Erste Komponente:* | | | | | | | |
| Katalysator **Beispiel 2** | 0,71 | - | - | - | - | - | - |
| Katalysator **Beispiel 11** | - | 0,48 | - | - | - | - | - |
| $MoO_2(acac)_2$[a] | - | - | 1,64 | - | - | - | - |
| Molybdäncarboxylat[b] | - | - | - | 0,70 | - | - | - |
| DBTDL[c] | - | - | - | - | 0,49 | - | - |
| Coscat® 83[d] | - | - | - | - | - | 0,02 | - |
| DABCO 33-LV®[e] | - | - | - | - | - | - | 0,14 |
| mmol-Equiv./100g[f] | 0,81 | 0,55 | 2,54 | 1,96 | 0,14 | 0,03 | 1,50 |
| *Zweite Komponente:* | | | | | | | |
| Desmodur® CD-L | 5,00 | 5,00 | 5,00 | 5,00 | 5,00 | 5,00 | 5,00 |

**Tabelle 4:** **Zweikomponentige Polyurethan-Zusammensetzungen** (Mengen in Gewichtsteilen).
[a] 28,6%ige Suspension in Methylethylketon. [b] Molybdän-2-ethylhexanoat (15,5% Mo, von Shepherd). [c] 10%ige Lösung von Dibutylzinn-dilaurat in Diisodecylphthalat. [d] Bismut-tris(neodecanoat) in Neodecansäure (16% Bi, von Erbslöh). [e] 33%ige Lösung von 1,4-Diazabicyclo[2.2.2]-octan in Dipropylenglykol (von Air Products). [f] mmol-Equivalente Metallatome bzw. Aminogruppen des Katalysators auf 100 g der Zusammensetzung.

Die Polyurethan-Zusammensetzungen wurden wie für Beispiel 27 beschrieben auf Aspekt, Zeit bis zur Klebefreiheit, Blasenbildung sowie Shore A-Härte geprüft, und zwar jeweils sowohl für die Zusammensetzung mit der frisch hergestellten ersten Komponente als auch für die Zusammensetzung mit der während 7 Tagen bei 60 °C gelagerten ersten Komponente. Ausschließlich für die Zusammensetzung mit der frisch hergestellten ersten Komponente wurde weiterhin wie für Beispiel 27 beschrieben die mechanischen Eigenschaften im Zugversuch gemessen, und zwar vor und nach verschiedenen Lagerungen zur beschleunigten Alterung der Proben.

Die Ergebnisse dieser Prüfungen sind in der **Tabelle 5** aufgeführt.

| Beispiel | 29 | 30 | V8 | V9 | V10 | V11 | V12 |
|---|---|---|---|---|---|---|---|
| *Zusammensetzung mit frisch hergestellter erster Komponente:* | | | | | | | |
| Aspekt | klar | klar | inh. | klar | klar | klar | klar |
| Hautbildungszeit (min.) | 15 | 35 | 17 | 105 | 27 | 90 | 35 |
| Shore A-Härte | 44 | 41 | 42 | 32 | 48 | 42 | 33 |
| Blasenbildung | keine | keine | keine | einige | viele | keine | viele |
| Zugfestigkeit (MPa): 7d/NK | 0,75 | 0,72 | 0,77 | 0,63 | 0,77 | 0,71 | 0,65 |
| +10d/Kataplasma | 0,73 | 0,71 | 0,77 | 0,59 | 0,77 | 0,73 | 0,66 |
| +1d/100°C | 0,77 | 0,75 | 0,76 | 0,62 | 0,48 | 0,70 | 0,72 |
| +10d/Kataplasma+1d/100°C | 0,75 | 0,72 | 0,87 | 0,66 | 0,52 | 0,74 | 0,69 |
| Bruchdehnung (%): 7d/NK | 97 | 117 | 115 | 109 | 105 | 124 | 135 |
| +10d/Kataplasma | 95 | 125 | 116 | 112 | 105 | 119 | 148 |
| +1d/100°C | 136 | 188 | 148 | 181 | 341 | 137 | 193 |
| +10d/Kataplasma+1d/100°C | 122 | 139 | 171 | 156 | 303 | 178 | 181 |
| E-Modul (MPa): 7d/NK | 1,42 | 0,99 | 1,12 | 0,89 | 1,20 | 0,82 | 0,88 |
| +10d/Kataplasma | 1,18 | 0,94 | 1,06 | 0,84 | 1,30 | 0,98 | 0,81 |
| +1d/100°C | 1,01 | 0,76 | 0,98 | 0,61 | 0,20 | 0,91 | 0,69 |
| +10d/Kataplasma+1d/100°C | 1,08 | 0,96 | 1,04 | 0,82 | 0,28 | 0,80 | 0,65 |

(fortgesetzt)

| Zusammensetzung mit gelagerter erster Komponente: | | | | | | | |
|---|---|---|---|---|---|---|---|
| Aspekt | klar | klar | inh. | klar | klar | klar | klar |
| Hautbildungszeit (min.) | 8 | 30 | 14 | 100 | 27 | 300 | 35 |
| Shore A-Härte | 45 | 44 | 45 | 40 | 45 | 41 | 40 |
| Blasenbildung | keine | keine | keine | einige | viele | einige | viele |
| **Tabelle 5: Eigenschaften der zweikomp. Polyurethan-Zusammensetzungen** | | | | | | | |

Aus der Tabelle 5 ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und blasenfrei zu einem Material mit vergleichsweise hoher Festigkeit und guter Beständigkeit aushärten.

**Beispiele 31 bis 39**

Wie für Beispiel 27 beschrieben wurden zur Herstellung der ersten Komponente jeweils ein Polyethertriol (Voranol® CP 4755, von Dow) und ein Katalysator gemäß Tabelle 6 vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die für Beispiel 27 beschriebene Weise mit einem modifizierten, bei Raumtemperatur flüssigen Diphenylmethandiisocyanat (Desmodur® CD-L, von Bayer) als zweite Komponente gemäß Tabelle 6 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 6 auf die gleiche Weise zu einer Polyurethan-Zusammensetzung vermischt.

Die Polyurethan-Zusammensetzungen wurden wie für Beispiel 27 beschrieben auf Aspekt, Zeit bis zur Klebefreiheit, Blasenbildung, Shore A-Härte sowie die mechanischen Eigenschaften im Zugversuch geprüft.

Die Ergebnisse dieser Prüfungen sind in der **Tabelle 7** aufgeführt.

| Beispiel | **31** | **32** | **33** | **34** | **35** | **36** | **37** | **38** | **39** |
|---|---|---|---|---|---|---|---|---|---|
| *Erste Komponente:* | | | | | | | | | |
| Voranol® CP 4755 | 30 | 30 | 30 | 30 | 30 | 30 | 30 | 30 | 30 |
| Katalysator **Beispiel 2** | 1,13 | 0,16 | - | - | - | - | - | - | - |
| Katalysator **Beispiel 5** | - | - | 1,22 | - | - | - | - | - | - |
| Katalysator **Beispiel 7** | - | - | - | 1,28 | - | - | - | - | - |
| Katalysator **Beispiel 10** | - | - | - | - | 0,46 | - | - | - | - |
| Katalysator **Beispiel 11** | - | - | - | - | - | 1,10 | - | - | - |
| Katalysator **Beispiel 14** | - | - | - | - | - | - | 0,39 | - | - |
| Katalysator **Beispiel 17** | - | - | - | - | - | - | - | 1,12 | - |
| Katalysator **Beispiel 22** | - | - | - | - | - | - | - | - | 0,35 |
| mmol-Equiv./100g[a] | 2,09 | 0,30 | 2,06 | 2,06 | 2,06 | 2,06 | 1,99 | 2,06 | 1,98 |
| *Zweite Komponente:* | | | | | | | | | |
| Desmodur® CD-L | 3,10 | 3,10 | 3,10 | 3,10 | 3,10 | 3,10 | 3,10 | 3,10 | 3,10 |
| **Tabelle 6: Zweikomponentige Polyurethan-Zusammensetzungen**<br>a mmol-Equivalente Molybdänatome des Katalysators auf 100 g der Zusammensetzung. | | | | | | | | | |

| Beispiel | **31** | **32** | **33** | **34** | **35** | **36** | **37** | **38** | **39** |
|---|---|---|---|---|---|---|---|---|---|
| *Zusammensetzung mit frisch hergestellter erster Komponente:* | | | | | | | | | |
| Aspekt | klar | klar | klar | klar | klar | klar | klar | klar | klar |
| Hautbildungszeit (min.) | 11 | 40 | 4 | 10 | 8 | 9 | 7 | 12 | 11 |
| Shore A-Härte | 32 | 39 | 44 | 44 | 45 | 42 | 48 | 46 | 45 |
| Blasenbildung | keine | keine | keine | keine | keine | keine | keine | keine | keine |
| *Zusammensetzung mit gelagerter erster Komponente:* | | | | | | | | | |
| Aspekt | klar | klar | klar | klar | klar | klar | klar | klar | klar |
| Hautbildungszeit (min.) | 7 | 31 | 5 | 5 | 3 | 8 | 6 | 6 | 2 |
| Shore A-Härte | 39 | 40 | 46 | 44 | 48 | 47 | 46 | 46 | 44 |
| Blasenbildung | keine | keine | keine | keine | keine | einige | keine | keine | keine |
| **Tabelle 7: Eigenschaften der zweikomponentigen Polyurethan-Zusammensetzungen.** | | | | | | | | | |

Aus der **Tabelle 7** ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und weitgehend blasenfrei zu einem Material mit guter Shore A-Härte aushärten.

**Patentansprüche**

1. Dioxomolybdän(VI)-Komplexverbindung der Formel $MoO_2(L)_x(Y)_{2-x}$, wobei x für 1 oder 2 steht, Y für einen einfach negativ geladenen Liganden steht und L für einen Liganden der Formel (I) steht,

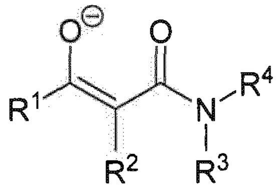

(I)

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen, wobei es sich bei der Dioxomolybdän(VI)-Komplexverbindung der Formel $MoO_2(L)_x(Y)_{2-x}$ nicht um Dioxomolybdän(VI)-bis(N,N-Diethyl-3-oxobutanamidat) handelt.

2. Dioxomolybdän(VI)-Komplexverbindung nach Anspruch 1, wobei $R^1$ für einen Alkyl-Rest mit 1 bis 4 Kohlenstoffatomen, einen Phenyl-Rest oder zusammen mit $R^2$ für einen zweiwertigen Alkylen-Rest mit 3 bis 4 Kohlenstoffatomen steht.

3. Dioxomolybdän(VI)-Komplexverbindung nach mindestens einem der vorangegangenen Ansprüche, wobei $R^2$ für einen Wasserstoff-Rest steht.

4. Dioxomolybdän(VI)-Komplexverbindung nach mindestens einem der vorangegangenen Ansprüche, wobei $R^3$ für einen Wasserstoff-Rest, einen Alkyl-Rest mit 1 bis 8 Kohlenstoffatomen, einen Cycloalkyl-Rest mit 5 bis 6 Kohlenstoffatomen, einen Hydroxyalkyl-Rest mit 1 bis 4 Kohlenstoffatomen, einen Alkylether-Rest mit 1 bis 4 Kohlenstoff-

atomen, oder zusammen mit $R^4$ für einen zweiwertigen Alkylen-Rest der Formel-$(CH_2)_n$-X-$(CH_2)_n$ - mit X= O, NR, wobei R ein einwertiger Alkyl-Rest mit 1 bis 4 Kohlenstoffatomen ist, oder S und n= 2 bis 6 steht.

**5.** Dioxomolybdän(VI)-Komplexverbindung nach mindestens einem der vorangegangenen Ansprüche, wobei $R^4$ für einen Wasserstoff-Rest, einen Alkyl-Rest mit 1 bis 8 Kohlenstoffatomen, einen Cycloalkyl-Rest mit 5 bis 6 Kohlenstoffatomen, einen Hydroxyalkyl-Rest mit 1 bis 4 Kohlenstoffatomen oder einen Alkylether-Rest mit 1 bis 4 Kohlenstoffatomen steht.

**6.** Dioxomolybdän(VI)-Komplexverbindung nach mindestens einem der vorangegangenen Ansprüche, wobei x für 2 steht.

**7.** Verfahren zur Herstellung der Dioxomolybdän(VI)-Komplexverbindung nach einem der Ansprüche 1 bis 6, wobei ein 1,3 Ketoamid der Formel

1

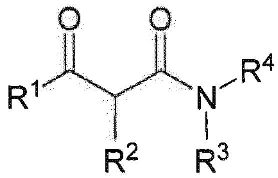

2

mit $R^1$, $R^2$, $R^3$ und $R^4$, wie vorstehend definiert, mit einem Dioxomolybdän(VI)-Salz oder -Komplex umgesetzt wird.

**8.** Verfahren nach Anspruch 7, wobei das Verhältnis zwischen dem Dioxomolybdän(VI)-Salz oder -Komplex und dem 1,3-Ketoamid im Bereich von 1:2 bis 1:4 liegt.

**9.** Verfahren nach Anspruch 7 oder 8, wobei als Dioxomolybdän(VI)-Komplex Dioxomolybdän(VI)-bis(acetylacetonat) eingesetzt wird.

**10.** Verwendung einer Dioxomolybdän(VI)-Komplexverbindung der Formel $MoO_2(L)_x(Y)_{2-x}$, wobei x für 1 oder 2 steht, Y für einen einfach negativ geladenen Liganden steht und L für einen Liganden der Formel (I) steht,

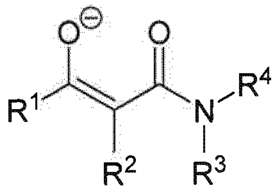

(I)

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen, als Katalysator für härtbare Massen, insbesondere für ein- oder zweikomponentige Polyurethan-Zusammensetzungen.

**11.** Zweikomponentige Polyurethan-Zusammensetzungen, umfassend mindestens ein Polyol als erste Komponente, mindestens ein Polyisocyanat als zweite Komponente und mindestens eine Dioxomolybdän(VI)-Komplexverbindung der Formel $MoO_2(L)_x(Y)_{2-x}$, wobei x für 1 oder 2 steht, Y für einen einfach negativ geladenen Liganden steht und L für einen Liganden der Formel (I) steht,

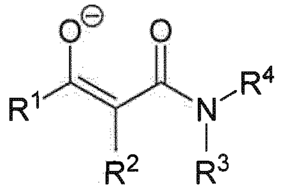

(I)

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

**12.** Zweikomponentige Polyurethan-Zusammensetzung nach Anspruch 11, wobei das Polyol ein Polyetherpolyol und das Polyisocyanat ein Diisocyanat ist.

**13.** Zweikomponentige Polyurethan-Zusammensetzung nach mindestens einem der Ansprüche 11 bis 12, wobei die Dioxomolybdän(VI)-Komplexverbindung 0,02 bis 10, bevorzugt 0,1 bis 5, und besonders bevorzugt 0,2 bis 3 mmol-Equivalente Molybdänatome auf 100 g der Zusammensetzung ausmacht.

**14.** Zweikomponentige Polyurethan-Zusammensetzung nach mindestens einem der Ansprüche 11 bis 13, wobei die Dioxomolybdän(VI)-Komplexverbindung in der ersten Komponente enthalten ist.

**15.** Einkomponentige Polyurethan-Zusammensetzung, umfassend mindestens ein Polyurethanprepolymer mit Isocyanatgruppen, hergestellt aus mindestens einem Polyisocyanat mit mindestens einem Polyol und mindestens eine Dioxomolybdän(VI)-Komplexverbindung der Formel $MoO_2(L)_x(Y)_{2-x}$, wobei x für 1 oder 2 steht, Y für einen einfach negativ geladenen Liganden steht und L für einen Liganden der Formel (I) steht,

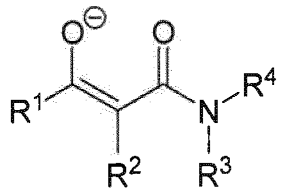

(I)

wobei $R^1$ und $R^2$ unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
$R^3$ und $R^4$ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

**16.** Verwendung der ein- oder zweikomponentigen Polyurethan-Zusammensetzung nach mindestens einem der Ansprüche 11 bis 15 als Vergussmasse, Dichtstoff, Klebstoff, Belag, Beschichtung, Lack, Voranstrich, Formteil, Elastomer für Bau- und Industrieanwendungen.

## Claims

**1.** Dioxomolybdenum(VI) complex compound of formula $MoO_2(L)_x(Y)_{2-x}$, where x stands for 1 or 2, Y for a ligand with a single negative charge, and L for a ligand of formula (I),

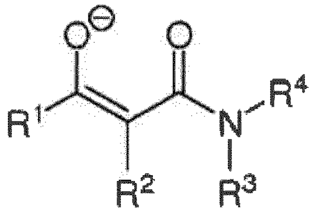

(I)

where $R^1$ and $R^2$ independently of one another stand for a hydrogen residue, for a monovalent saturated or unsaturated hydrocarbon residue having 1 to 10 carbon atoms, or together stand for a bivalent alkylene residue having 3 to 6 carbon atoms, and
$R^3$ and $R^4$ independently of one another stand for a hydrogen residue, a monovalent saturated hydrocarbon residue, which optionally contains heteroatoms, having 1 to 12 carbon atoms, or together stand for a bivalent alkylene residue, which optionally contains heteroatoms, having 3 to 6 carbon atoms, where the dioxomolybdenum(VI) complex compound of formula $MoO_2(L)_x(Y)_{2-x}$ is not dioxomolybdenum(VI) bis(N,N-diethyl-3-oxobutanamidate).

2. Dioxomolybdenum(VI) complex compound according to Claim 1, where $R^1$ stands for an alkyl residue having 1 to 4 carbon atoms, for a phenyl residue, or together with $R^2$ stands for a bivalent alkylene residue having 3 to 4 carbon atoms.

3. Dioxomolybdenum(VI) complex compound according to at least one of the previous claims, where $R^2$ stands for a hydrogen residue.

4. Dioxomolybdenum(VI) complex compound according to at least one of the previous claims, where $R^3$ stands for a hydrogen residue, an alkyl residue having 1 to 8 carbon atoms, a cycloalkyl residue having 5 to 6 carbon atoms, a hydroxyalkyl residue having 1 to 4 carbon atoms, an alkyl ether residue having 1 to 4 carbon atoms, or together with $R^4$ stands for a bivalent alkylene residue of formula $-(CH_2)_n-X-(CH_2)_n-$ with X = O, NR, where R is a monovalent alkyl residue having 1 to 4 carbon atoms, or S, and n = 2 to 6.

5. Dioxomolybdenum(VI) complex compound according to at least one of the previous claims, where $R^4$ stands for a hydrogen residue, an alkyl residue having 1 to 8 carbon atoms, a cycloalkyl residue having 5 to 6 carbon atoms, a hydroxyalkyl residue having 1 to 4 carbon atoms or an alkyl ether residue having 1 to 4 carbon atoms.

6. Dioxomolybdenum(VI) complex compound according to at least one of the previous claims, where x stands for 2.

7. Method for preparing the dioxomolybdenum(VI) complex compound according to one of Claims 1 to 6, wherein a 1,3-ketoamide of formula

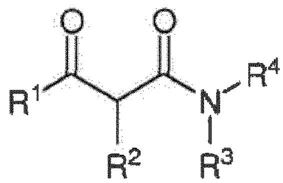

1

2

with $R^1$, $R^2$, $R^3$ and $R^4$, as defined above, is reacted with a dioxomolybdenum(VI) salt or complex.

8. Method according to Claim 7, wherein the ratio between the dioxomolybdenum(VI) salt or complex and the 1,3-ketoamide is in the range from 1:2 to 1:4.

9. Method according to Claim 7 or 8, wherein, as dioxomolybdenum(VI) complex, dioxomolybdenum(VI) bis(acetylacetonate) is used.

10. Use of a dioxomolybdenum(VI) complex compound of formula $MoO_2(L)_x(Y)_{2-x}$, where x stands for 1 or 2, Y for a ligand with a single negative charge, and L for a ligand of formula (I),

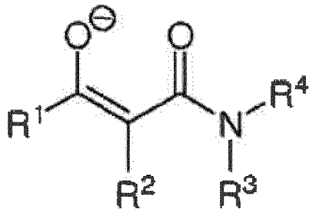

(I)

where $R^1$ and $R^2$ independently of one another stand for a hydrogen residue, for a monovalent saturated or unsaturated hydrocarbon residue having 1 to 10 carbon atoms, or together stand for a bivalent alkylene residue having 3 to 6 carbon atoms, and
$R^3$ and $R^4$ independently of one another stand for a hydrogen residue, a monovalent saturated hydrocarbon residue, which optionally contains heteroatoms, having 1 to 12 carbon atoms, or together stand for a bivalent alkylene residue, which optionally contains heteroatoms, having 3 to 6 carbon atoms, as catalyst for curable compositions, in particular for single-component or two-component polyurethane compositions.

**11.** Two-component polyurethane compositions comprising at least one polyol as first component, at least one polyisocyanate as second component, and at least one dioxomolybdenum(VI) complex compound of formula $MoO_2(L)_n(Y)_{2-x}$, where x stands for 1 or 2, Y for a ligand with a single negative charge, and L for a ligand of formula (I),

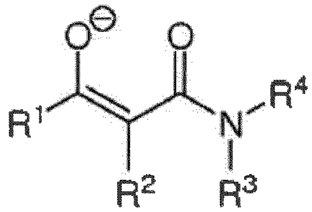

(I)

where $R^1$ and $R^2$ independently of one another stand for a hydrogen residue, for a monovalent saturated or unsaturated hydrocarbon residue having 1 to 10 carbon atoms, or together stand for a bivalent alkylene residue having 3 to 6 carbon atoms, and
$R^3$ and $R^4$ independently of one another stand for a hydrogen residue, a monovalent saturated hydrocarbon residue, which optionally contains heteroatoms, having 1 to 12 carbon atoms, or together stand for a bivalent alkylene residue, which optionally contains heteroatoms, having 3 to 6 carbon atoms.

**12.** Two-component polyurethane composition according to Claim 11, wherein the polyol is a polyether polyol and the polyisocyanate is a diisocyanate.

**13.** Two-component polyurethane composition according to at least one of Claims 11 to 12, wherein the dioxomolybdenum(VI) complex compound represents 0.02 to 10, preferably 0.1 to 5, and particularly preferably 0.2 to 3 mmol equivalents of molybdenum atoms per 100 g of the composition.

**14.** Two-component polyurethane composition according to at least one of Claims 11 to 13, wherein the dioxomolybdenum(VI) complex compound is contained in the first component.

**15.** Single-component polyurethane composition comprising at least one polyurethane prepolymer with isocyanate groups, prepared from at least one polyisocyanate with at least one polyol and at least one dioxomolybdenum(VI) complex compound of formula $MoO_2(L)_x(Y)_{2-x}$, where x stands for 1 or 2, Y for a ligand with a single negative charge, and L for a ligand of formula (I),

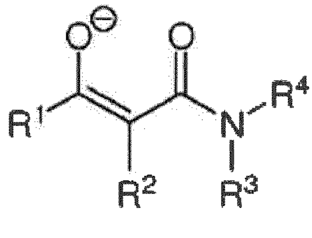

(I)

where $R^1$ and $R^2$ independently of one another stand for a hydrogen residue, for a monovalent saturated or unsaturated hydrocarbon residue having 1 to 10 carbon atoms, or together stand for a bivalent alkylene residue having 3 to 6 carbon atoms, and
$R^3$ and $R^4$ independently of one another stand for a hydrogen residue, a monovalent saturated hydrocarbon residue, which optionally contains heteroatoms, having 1 to 12 carbon atoms, or together stand for a bivalent alkylene residue, which optionally contains heteroatoms, having 3 to 6 carbon atoms.

**16.** Use of the single-component or two-component polyurethane composition according to at least one of Claims 11 to 15 as casting composition, sealant, adhesive, covering, coating, paint, primer, molded part, elastomer for applications in construction and industry.

## Revendications

**1.** Composé complexe de dioxomolybdène (VI) de formule $MoO_2(L)_x(Y)_{2-x}$, x représentant 1 ou 2, Y représentant un ligand à une charge négative et L représentant un ligand de formule (I)

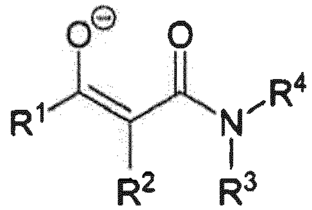 (I)

dans laquelle $R^1$ et $R^2$ représentent indépendamment l'un de l'autre un radical hydrogène, un radical hydrocarboné monovalent saturé ou insaturé de 1 à 10 atomes de carbone, ou représentent ensemble un radical alkylène bivalent de 3 à 6 atomes de carbone, et
$R^3$ et $R^4$ représentent indépendamment l'un de l'autre un radical hydrogène, un radical hydrocarboné monovalent saturé, qui contient éventuellement des hétéroatomes, de 1 à 12 atomes de carbone, ou représentent ensemble un radical alkylène bivalent, qui contient éventuellement des hétéroatomes, de 3 à 6 atomes de carbone, le composé complexe de dioxomolybdène (VI) de formule $MoO_2(L)_x(Y)_{2-x}$ n'étant pas le bis(N,N-diéthyl-3-oxobutanamidate) de dioxomolybdène (VI).

**2.** Composé complexe de dioxomolybdène (VI) selon la revendication 1, dans lequel $R^1$ représente un radical alkyle de 1 à 4 atomes de carbone, un radical phényle ou représente conjointement avec $R^2$ un radical alkylène bivalent de 3 à 4 atomes de carbone.

**3.** Composé complexe de dioxomolybdène (VI) selon au moins l'une quelconque des revendications précédentes, dans lequel $R^2$ représente un radical hydrogène.

**4.** Composé complexe de dioxomolybdène (VI) selon au moins l'une quelconque des revendications précédentes, dans lequel $R^3$ représente un radical hydrogène, un radical alkyle de 1 à 8 atomes de carbone, un radical cycloalkyle de 5 à 6 atomes de carbone, un radical hydroxyalkyle de 1 à 4 atomes de carbone, un radical alkyléther de 1 à 4 atomes de carbone, ou représente conjointement avec $R^4$ un radical alkylène bivalent de formule $-(CH_2)_n-X-(CH_2)_n-$ avec X = O, NR, R étant un radical alkyle monovalent de 1 à 4 atomes de carbone, ou S et n = 2 à 6.

**5.** Composé complexe de dioxomolybdène (VI) selon au moins l'une quelconque des revendications précédentes, dans lequel $R^4$ représente un radical hydrogène, un radical alkyle de 1 à 8 atomes de carbone un radical cycloalkyle de 5 à 6 atomes de carbone, un radical hydroxyalkyle de 1 à 4 atomes de carbone, ou un radical alkyléther de 1 à 4 atomes de carbone.

**6.** Composé complexe de dioxomolybdène (VI) selon au moins l'une quelconque des revendications précédentes, dans lequel x représente 2.

**7.** Procédé de fabrication du composé complexe de dioxomolybdène (VI) selon l'une quelconque des revendications 1 à 6, dans lequel un 1,3-cétoamide de formule

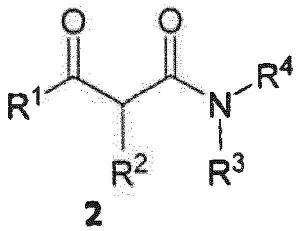

1

2

avec $R^1$, $R^2$, $R^3$ et $R^4$ tels que définis précédemment, est mis en réaction avec un sel ou complexe de dioxomolybdène (VI).

8. Procédé selon la revendication 7, dans lequel le rapport entre le sel ou complexe de dioxomolybdène (VI) et le 1,3-cétoamide se situe dans la plage allant de 1:2 à 1:4.

9. Procédé selon la revendication 7 ou 8, dans lequel le bis(acétylacétonate) de dioxomolybdène (VI) est utilisé en tant que complexe de dioxomolybdène (VI).

10. Utilisation d'un composé complexe de dioxomolybdène (VI) de formule $MoO_2(L)_x(Y)_{2-x}$, x représentant 1 ou 2, Y représentant un ligand à une charge négative et L représentant un ligand de formule (I)

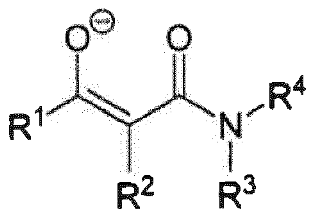

(I)

dans laquelle $R^1$ et $R^2$ représentent indépendamment l'un de l'autre un radical hydrogène, un radical hydrocarboné monovalent saturé ou insaturé de 1 à 10 atomes de carbone, ou représentent ensemble un radical alkylène bivalent de 3 à 6 atomes de carbone, et
$R^3$ et $R^4$ représentent indépendamment l'un de l'autre un radical hydrogène, un radical hydrocarboné monovalent saturé, qui contient éventuellement des hétéroatomes, de 1 à 12 atomes de carbone, ou représentent ensemble un radical alkylène bivalent, qui contient éventuellement des hétéroatomes, de 3 à 6 atomes de carbone, en tant que catalyseur pour matériaux durcissables, notamment pour compositions de polyuréthane mono- ou bicomposantes.

11. Compositions de polyuréthane bicomposantes, comprenant au moins un polyol en tant que premier composant, au moins un polyisocyanate en tant que second composant et au moins un composé complexe de dioxomolybdène (VI) de formule $MoO_2(L)_x(Y)_{2-x}$, x représentant 1 ou 2, Y représentant un ligand à une charge négative et L représentant un ligand de formule (I)

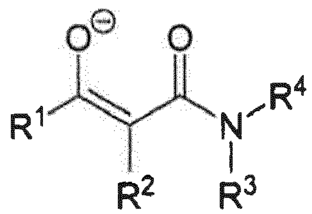

(I)

dans laquelle $R^1$ et $R^2$ représentent indépendamment l'un de l'autre un radical hydrogène, un radical hydrocarboné monovalent saturé ou insaturé de 1 à 10 atomes de carbone, ou représentent ensemble un radical alkylène bivalent de 3 à 6 atomes de carbone, et
$R^3$ et $R^4$ représentent indépendamment l'un de l'autre un radical hydrogène, un radical hydrocarboné monovalent saturé, qui contient éventuellement des hétéroatomes, de 1 à 12 atomes de carbone, ou représentent ensemble un radical alkylène bivalent, qui contient éventuellement des hétéroatomes, de 3 à 6 atomes de carbone.

**12.** Composition de polyuréthane bicomposante selon la revendication 11, dans laquelle le polyol est un polyéther-polyol et le polyisocyanate est un diisocyanate.

**13.** Composition de polyuréthane bicomposante selon au moins l'une quelconque des revendications 11 à 12, dans laquelle le composé complexe de dioxomolybdène (VI) représente 0,02 à 10, de préférence 0,1 à 5, et de manière particulièrement préférée 0,2 à 3 équivalents mmol d'atomes de molybdène pour 100 g de la composition.

**14.** Composition de polyuréthane bicomposante selon au moins l'une quelconque des revendications 11 à 13, dans laquelle le composé complexe de dioxomolybdène (VI) est contenu dans le premier composant.

**15.** Composition de polyuréthane monocomposante, comprenant au moins un prépolymère de polyuréthane à groupes isocyanate, fabriqué à partir d'au moins un polyisocyanate avec au moins un polyol et au moins un composé complexe de dioxomolybdène (VI) de formule $MoO_2(L)_x(Y)_{2-x}$, x représentant 1 ou 2, Y représentant un ligand à une charge négative et L représentant un ligand de formule (I)

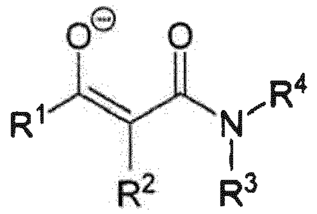

(I)

dans laquelle $R^1$ et $R^2$ représentent indépendamment l'un de l'autre un radical hydrogène, un radical hydrocarboné monovalent saturé ou insaturé de 1 à 10 atomes de carbone, ou représentent ensemble un radical alkylène bivalent de 3 à 6 atomes de carbone, et
$R^3$ et $R^4$ représentent indépendamment l'un de l'autre un radical hydrogène, un radical hydrocarboné monovalent saturé, qui contient éventuellement des hétéroatomes, de 1 à 12 atomes de carbone, ou représentent ensemble un radical alkylène bivalent, qui contient éventuellement des hétéroatomes, de 3 à 6 atomes de carbone.

**16.** Utilisation de la composition de polyuréthane mono- ou bicomposante selon au moins l'une quelconque des revendications 11 à 15 en tant que matériau de scellement, matériau d'étanchéité, adhésif, garniture, revêtement, vernis, apprêt, pièce moulée, élastomère pour des applications dans le bâtiment et l'industrie.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10131463 A1
- DE 10308104
- WO 2009106722 A1
- DE 102005041246
- WO 2007003966 A
- EP 1408062 A1
- EP 1408062 A


### In der Beschreibung aufgeführte Nicht-Patentliteratur


- *Inorganica Chimica Acta,* Januar 1981, vol. 56 (2), L27-L28